# EUROPEAN PATENT APPLICATION

(11) **EP 4 767 962 A1**
(43) Date of publication of application: **01.07.2026**
(21) Application number: 25220778.2
(22) Date of filing: 04.12.2025
(51) Int. Cl.: A61B 18/12

(54) **CATHETER WITH PULSED FIELD ABLATION ELECTRODES**

(30) Priority: 27.12.2024 US 202463739156 P; 17.11.2025 US 202519390828
(71) Applicant: Biosense Webster (Israel) Ltd., 2066717 Yokneam (IL)
(72) Inventor: SUAREZ, Paul A., Irvine, 92618 (US); HIGHSMITH, Debby E., Irvine, 92618 (US); BASU, Shubhayu, Irvine, 92618 (US); BERGER, Abraham, 2066717 Yokneam (IL); BERGER, Omer, 2066717 Yokneam (IL); BAR-TAL, Meir, 2066717 Yokneam (IL); OSADCHY, Daniel, 2066717 Yokneam (IL)
(74) Representative: Carpmaels & Ransford LLP

(57) **Abstract**

An apparatus includes an elongate shaft assembly and a processor. The elongate shaft assembly includes a distal portion having an end effector and a tip member. The end effector has a flex circuit with a plurality of electrodes extending angularly about the distal portion of the shaft assembly and spaced longitudinally apart from each other along the distal portion of the shaft assembly. The tip member is positioned distal to the end effector and is more flexible than the distal portion of the shaft assembly. The processor is electrically coupled with the flex circuit and is configured to select one or more electrodes of the plurality of electrodes to activate. The processor is further configured to activate the one or more selected electrodes to apply electrical energy to tissue to thereby ablate the tissue.

## Description

### PRIORITY

This application claims priority to U.S. Provisional Pat. App. No. 63/739,156, entitled "Ablation Catheter with Longitudinally Spaced Electrodes," filed December 27, 2024, the disclosure of which is incorporated by reference herein, in its entirety.

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively applying electrical energy to cardiac tissue, it may be possible to cease or modify the propagation of unwanted electrical signals from one portion of the heart to another. Some such ablation treatments may include radio frequency (RF) ablation with alternating current (AC) electrical energy; and/or irreversible electroporation (IRE) via pulsed field direct current (DC) electrical energy (e.g., pulsed field ablation (PFA)). The ablation process may provide a barrier to unwanted electrical pathways by creating electrically insulative lesions or scar tissue that effectively block communication of aberrant electrical signals across the ablated tissue.

In some procedures, a catheter with one or more electrodes may be used to provide ablation within a patient. The catheter may be inserted into a major vein or artery (e.g., the femoral artery) and then advanced to position the electrodes within the heart or in a structure adjacent to the heart (e.g., the pulmonary vein). The one or more electrodes may be placed in contact with cardiac tissue or other vascular tissue and then activated with electrical energy to thereby ablate the contacted tissue (e.g., via RF energy, IRE, etc.). In some cases, the electrodes may be bipolar. In some other cases, a monopolar electrode may be used in conjunction with a ground pad or other reference electrode that is in contact with the patient. Irrigation may be used to draw heat from components of an ablation catheter; and to prevent the formation of blood clots near the tissue treatment site.

Examples of ablation catheters are described in U.S. Pat. No. 8,747,351, entitled "Catheter with Multi-Functional Control Handle Having Linear Mechanism," issued June 10, 2014, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; U.S. Pat. No. 9,220,433, entitled "Catheter with Variable Arcuate Distal Section," issued December 29, 2015, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 10,702,177, entitled "Catheter with Bipole Electrode Spacer and Related Methods," issued July 7, 2020, the disclosure of which is incorporated by reference herein, in its entirety; U.S. Pat. No. 10,743,932, entitled "Integrated Ablation System using Catheter with Multiple Irrigation Lumens," issued August 18, 2020, the disclosure of which is incorporated by reference herein, in its entirety; and U.S. Pat. No. 11,559,349, entitled "Ablation Catheter with a Flexible Printed Circuit Board," issued January 24, 2023, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pat. No. 10,702,177, entitled "Catheter with Bipole Electrode Spacer and Related Methods," issued July 7, 2020, the disclosure of which is incorporated by reference herein, in its entirety.

Some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient.
FIG. 2 depicts a perspective view of an example of a catheter assembly that may be used in the medical procedure of FIG. 1.
FIG. 3 depicts a perspective view of a distal portion of a shaft assembly of the catheter assembly of FIG. 2.
FIG. 4 depicts a perspective view of a distal portion of a flex circuit of the catheter assembly of FIG. 2.
FIG. 5 depicts a perspective view of a proximal portion of the flex circuit of FIG. 4.
FIG. 6 depicts a cross-sectional side view of a distal portion of the catheter assembly of FIG. 2, with an outer layer omitted from the shaft assembly.
FIG. 7 depicts a cross-sectional end view of the shaft assembly of FIG. 3.
FIG. 8 depicts a bottom plan view of the flex circuit of FIG. 4, with the flex circuit in a flat configuration.
FIG. 9 depicts a top plan view of a distal portion of the flex circuit of FIG. 4, with the flex circuit in a flat configuration.
FIG. 10 depicts a bottom plan view of a proximal portion of the flex circuit of FIG. 4, with the flex circuit in a flat configuration.
FIG. 11 depicts a side elevation view of the flex circuit of FIG. 4, with the flex circuit in a wrapped configuration.
FIG. 12 depicts a side elevation view of a distal portion of the flex circuit of FIG. 4, with the flex circuit in a wrapped configuration.
FIG. 13 depicts a top plan view of a distal portion of the flex circuit of FIG. 4, with the flex circuit in a wrapped configuration.
FIG. 14 depicts a side elevation view of a proximal portion of the flex circuit of FIG. 4, with the flex circuit in a wrapped configuration.
FIG. 15 depicts a top plan view of a proximal portion of the flex circuit of FIG. 4, with the flex circuit in a wrapped configuration.
FIG. 16A depicts a schematic view of a catheter shaft with a first example of an elongate position sensor arrangement, with a position sensor loop in a non-bent state.
FIG. 16B depicts a schematic view of the position sensor loop of FIG. 16A in an example of a bent state.
FIG. 16C depicts a schematic top view of the position sensor loop of FIG. 16A in the non-bent state of FIG. 16A.
FIG. 16D depicts a schematic top view of the position sensor loop of FIG. 16A in the bent state of FIG. 16B.
FIG. 17 depicts a schematic view of a catheter shaft with a second example of an elongate position sensor arrangement.
FIG. 18 depicts a bottom plan view of an example of a flex circuit that may be used to provide the elongate position sensor arrangement of FIG. 17.
FIG. 19 depicts a bottom plan view of an intermediate portion of the flex circuit of FIG. 18.
FIG. 20 depicts a schematic view of a catheter shaft with a third example of an elongate position sensor arrangement.
FIG. 21 depicts a schematic view of a catheter shaft with a fourth example of an elongate position sensor arrangement.
FIG. 22 depicts a schematic view of the end effector of the catheter assembly of FIG. 2 with electrodes of the end effector being used with a ground pad in a first example of an electrical path.
FIG. 23 depicts a schematic view of the end effector of the catheter assembly of FIG. 2 with electrodes of the end effector being used in a second example of an electrical path.
FIG. 24 depicts a schematic view of the end effector of the catheter assembly of FIG. 2 with electrodes of the end effector being used in a third example of an electrical path.
FIG. 25 depicts a schematic view of the end effector of the catheter assembly of FIG. 2 with electrodes of the end effector being used in a fourth example of an electrical path.
FIG. 26 depicts a schematic view of the end effector of the catheter assembly of FIG. 2 with electrodes of the end effector being used in a fifth example of an electrical path.
FIG. 27 depicts a schematic view of the end effector of the catheter assembly of FIG. 2 with electrodes of the end effector being used in a sixth example of an electrical path.
FIG. 28 depicts a schematic view of the end effector of the catheter assembly of FIG. 2 with electrodes of the end effector being used with a guidewire in a seventh example of an electrical path.
FIG. 29 depicts a schematic view of the end effector of the catheter assembly of FIG. 2 with electrodes of the end effector being used with a guidewire in an eighth example of an electrical path.
FIG. 30 depicts a schematic view of the end effector of the catheter assembly of FIG. 2 with electrodes of the end effector being used with irrigation fluid from the catheter assembly in a ninth example of an electrical path.
FIG. 31 depicts a schematic view of the end effector of the catheter assembly of FIG. 2 with electrodes of the end effector being used with irrigation fluid from a sheath in a tenth example of an electrical path.
FIG. 32 depicts a side elevation view of an example of a variation of the end effector of the catheter assembly of FIG. 2.
FIG. 32A depicts a schematic cross-sectional side view of a portion of the end effector of FIG. 32.
FIG. 33 depicts a schematic view of the end effector of the catheter assembly of FIG. 2 with electrodes of the end effector being used with another ablation instrument in an eleventh example of an electrical path.
FIG. 34 depicts a schematic view of the end effector of the catheter assembly of FIG. 2 with electrodes of the end effector being used with another ablation instrument in a twelfth example of an electrical path.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Example of Catheter System

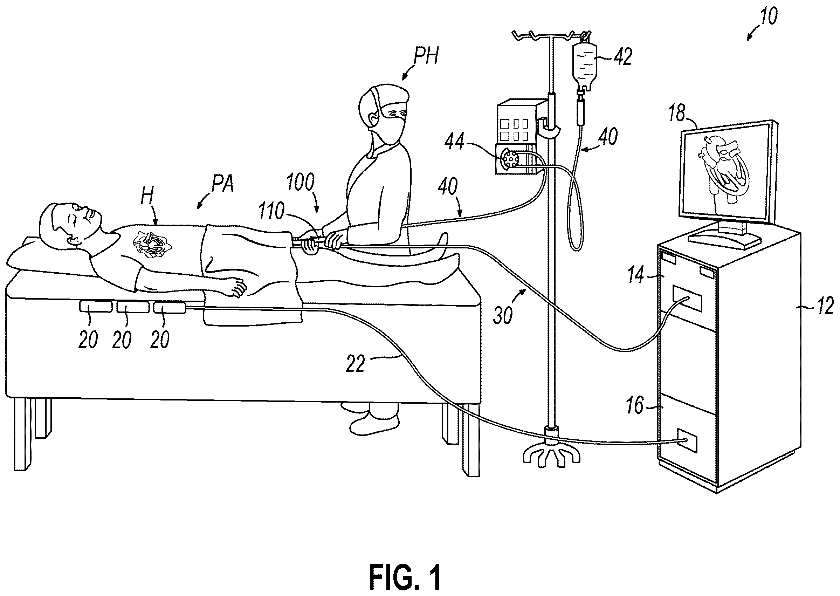

FIG. 1 shows an example of a medical procedure and associated components of a cardiac catheter system that may be used to provide EP mapping and/or cardiac ablation as referred to above. In particular, FIG. 1 shows a physician (PH) grasping a handle assembly (110) of a catheter assembly (100), with an end effector of a catheter (not shown) of catheter assembly (100) disposed in a patient (PA) to map potentials in tissue and/or ablate tissue in or near the heart (H) of the patient (PA). Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22).

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via electrodes of the end effector. In such versions. console (12) includes a processor (not shown) that processes such EP mapping signals and thereby provides EP mapping as is known in the art. In some other versions, the end effector does not provide EP mapping. In addition to receiving EP mapping signals obtained via electrodes of the end effector, or as an alternative to receiving EP mapping signals obtained via electrodes of the end effector, first driver module (14) may be operable to provide electrical power to electrodes of the end effector, to thereby ablate tissue. In such versions. console (12) includes a processor (not shown) that drives generation of the appropriate electrical signals to provide ablation as is known in the art. In still other versions, the end effector does not provide ablation.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) may also be operable to receive position indicative signals from one or more position sensors (not shown) in the end effector and/or elsewhere in the catheter. In such versions, the processor of console (12) is operable to process the position indicative signals from the one or more position sensors to thereby determine the position of the end effector within the patient (PA). In some versions, each position sensor includes one or more coils that are operable to generate signals that are indicative of the position and orientation of the end effector within the patient (PA). The coils are configured to provide electrical signals in response to the presence of an alternating electromagnetic field generated by field generators (20). Other components and techniques that may be used to generate real-time position data associated with the end effector may include wireless triangulation, acoustic tracking, optical tracking, inertial tracking, and the like. In some other variations, the end effector and/or the catheter lack a position sensor.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the position sensor assembly of the end effector and/or catheter. For instance, as the end effector of the catheter moves within the patient (PA), the corresponding position data from the one or more position sensors may cause the processor of console (12) to update the patient anatomy views in display (18) in real time to depict the regions of patient anatomy around the end effector as the end effector moves within the patient (PA). Moreover, the processor of console (12) may drive display (18) to show locations of aberrant conductive tissue sites, as detected via electrophysiological (EP) mapping with the end effector or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of the end effector on the images of the patient's anatomy, such as by superimposing an illuminated dot, a crosshair, a graphical representation of the end effector, or some other form of visual indication. Such a superimposed visual indication may also move within the images of the patient anatomy on display (18) in real time as the physician moves the end effector within the patient (PA), thereby providing real-time visual feedback to the operator about the position of the end effector within the patient (PA) as the end effector moves within the patient (PA). The images provided through display (18) may thus effectively provide a video tracking the position of the end effector within a patient (PA), without necessarily having any optical instrumentation (i.e., cameras) viewing the end effector. In the same view, display (18) may simultaneously visually indicate the locations of aberrant conductive tissue sites detected through EP mapping. The physician (PH) may thus view display (18) to observe the real time positioning of the end effector in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). Such irrigation fluid may be expelled through openings (not shown) of the end effector. Such irrigation may be provided in any suitable fashion as will be apparent to those skilled in the art in view of the teachings herein.

### II. Example of Catheter Assembly

As noted above, a catheter assembly (100) may be used to perform diagnostic functions (e.g., EP mapping, etc.) and/or therapeutic functions (e.g., ablation, etc.) within a cardiovascular system of a patient. In some cases, the end effector of catheter assembly (100) may be positioned in anatomical spaces such as chambers of the heart, the pulmonary vein, etc. to provide diagnostic and/or therapeutic functionality in those spaces. In addition, or in the alternative, it may be desirable to advance an end effector into smaller anatomical spaces, such as the Vein of Marshall and/or other small vessels branching out from the coronary sinus, to provide diagnostic and/or therapeutic functionality in those smaller spaces.

To the extent that some conventional EP mapping and/or ablation end effectors may fit within larger anatomical spaces such as chambers of the heart and the pulmonary vein, such end effectors may not fit within smaller anatomical spaces such as the Vein of Marshall and/or other small vessels branching out from the coronary sinus. Moreover, it may not be practical to simply scale down the size of such conventional end effectors to make them fit within such smaller spaces, such that a scaling-down in size is not a mere design choice. For instance, it may be physically impossible to simply reduce the size of electrodes and/or position sensors of such conventional end effectors to fit within smaller spaces. In cases where it is theoretically physically possible to simply reduce the size of electrodes and/or position sensors of such conventional end effectors to fit within smaller spaces, the scaling-down in the size of the electrodes and/or position sensors may adversely affect the functionality of the electrodes and/or position sensors. For instance, scaled-down electrodes may fail to provide adequate EP mapping and/or ablation functioning and scaled-down position sensors may fail to provide adequate position-indicating signals and accuracy. It may therefore be necessary to provide a new configuration and/or arrangement of electrodes and/or position sensors to provide the desired functionality within relatively small spaces such as the Vein of Marshall and/or other small vessels branching out from the coronary sinus.

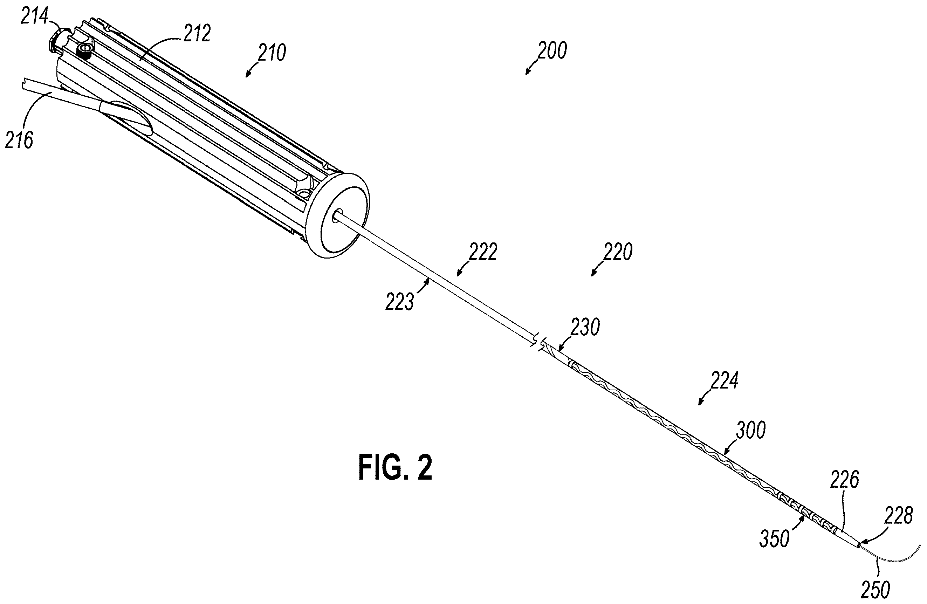

FIG. 2 shows an example of a catheter assembly (200) having an end effector (350) that may provide the desired EP mapping, ablation, and/or position sensing functionality within relatively small anatomical spaces such as the Vein of Marshall and/or other small vessels branching out from the coronary sinus. Catheter assembly (200) of this example includes a handle assembly (210) and a shaft assembly (220) extending distally from handle assembly (210).

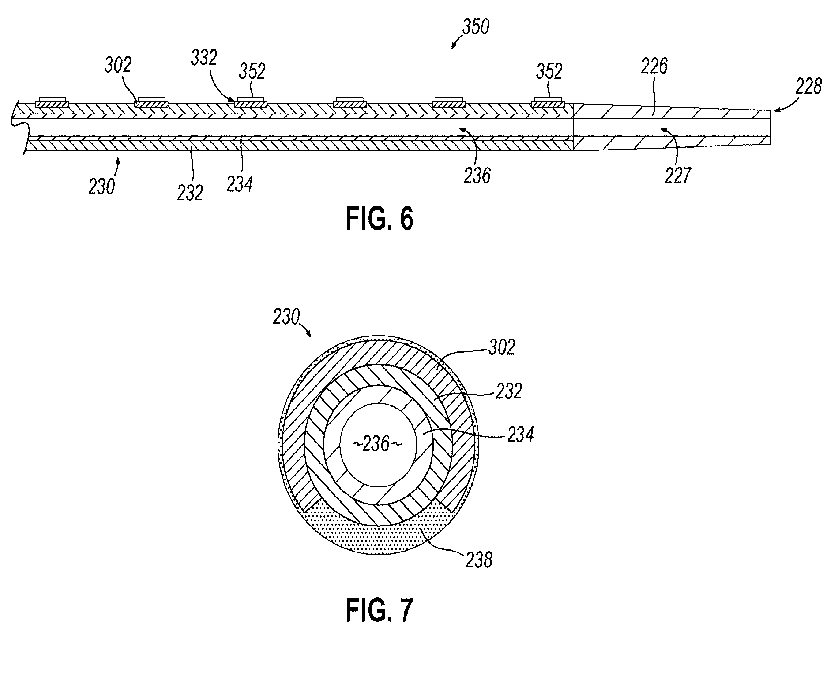

Handle assembly (210) includes a body (212), a port (214), and a cable (216). Body (212) is configured to be gripped by the hand of a physician (PH). In some versions, body (212) includes one or more actuators that are operable to drive movement of shaft assembly (220) (e.g., rotation, articulation, helical contraction, etc.) and/or other functionality. In some alternative versions, body (212) is configured to engage with a robotic arm or other automated assembly, rather than being operated by the hand of a physician (PH). Port (214) is positioned and configured to provide access to a lumen (236) (FIGS. 6-7) of shaft assembly (220), thereby allowing insertion of other components (e.g., a guidewire (250), etc.) into shaft assembly (220) and/or communication of fluid (e.g., irrigation fluid such as saline, ethanol, contrast medium, etc.) via shaft assembly (220). For instance, a fluid conduit, like fluid conduit (40) described above, may be coupled with port (214) in some scenarios. Cable (216) is configured to provide electrical communication between electrical components of catheter assembly (200) (e.g., flex circuit (300), described below) and guidance and drive system (10). Cable (216) may thus represent a version of cable (30) described above with reference to FIG. 1.

Shaft assembly (220) of this example includes a proximal portion (222) and a distal portion (224), with end effector (350) being provided at distal portion (224). Proximal portion (222) comprises a rigid shaft (223) in the present example. For instance, proximal portion (222) may comprise a metallic hypotube. Alternatively, proximal portion (222) may comprise any other suitable material(s). Distal portion (224) comprises a flexible catheter body (230). In some versions, a distal region of proximal portion (222) comprises laser cuts and/or other features that provide a gradual transition in stiffness between proximal portion (222) and distal portion (224). Examples of materials and configurations that may be used to form distal portion (224) will be described in greater detail below.

A tip member (226) is secured to the distal end of distal portion (224) of catheter body (230), distal to end effector (350). As shown in FIG. 2, a guidewire (250) may be slidably disposed in lumen (236) of catheter body (230), extending distally from tip member (226). In some versions, guidewire (250) comprises a nonferrous material (e.g., nitinol, SS316, etc.), though any suitable material(s) may be used to form guidewire (250). Guidewire (250) may be inserted into catheter assembly (200) via port (214). Similarly, guidewire (250) may be removed from catheter assembly (200) via port (214). In some versions, at least a portion of guidewire (250) has a predefined shape, and distal portion (224) of shaft assembly (220) conforms to this predefined shape of guidewire (250) when guidewire (250) is disposed in lumen (236). Alternatively, guidewire (250) may be flexible without a predefined shape, such that the positioning of guidewire (250) in lumen (236) does not impart a predefined shape to distal portion (224) of shaft assembly (220).

Shaft assembly (220) may have any suitable dimensions. By way of example only, shaft assembly (220) may have an outer diameter ranging from approximately 2 French (e.g., approximately 0.67 mm) to approximately 4 French (e.g., approximately 1.33 mm), or more particularly approximately 3 French (e.g., approximately 1.00 mm); or any other suitable outer diameter. By way of further example only, shaft assembly (220) may have an inner diameter ranging from approximately 0.25 mm to approximately 0.45 mm; or any other suitable inner diameter. Shaft assembly (220) may also have any suitable length. For instance, shaft assembly (220) may have a length ranging from approximately 150 cm to approximately 200 cm, or more specifically may be approximately 173 cm; or may be any other suitable length. By way of further example only, distal portion (224) may have a length ranging from approximately 30 cm to approximately 75 cm, or more specifically may be approximately 46 cm; or may be any other suitable length.

In some versions, a region of distal portion (224) is steerable or operable to articulate, such that end effector (350) may be controllably deflected laterally away from a central longitudinal axis. In some such versions, such steerability is provided in accordance with at least some of the teachings of U.S. Pat. No. 8,747,351, entitled "Catheter with multi-functional control handle having linear mechanism," issued June 10, 2014, the disclosure of which is incorporated by reference herein. In some other versions, catheter assembly (200) lacks a feature providing controlled deflection of shaft assembly (220). As another example, a region of distal portion (224) may be configured to controllably deform in a helical shape, with the radius and/or pitch being controllable by the physician (PH). In some such versions, such controllable contraction of distal portion (224) is provided in accordance with at least some of the teachings of U.S. Pat. No. 9,220,433, entitled "Catheter with Variable Arcuate Distal Section," issued December 29, 2015, the disclosure of which is incorporated by reference herein, in its entirety. In some other versions, catheter assembly (100) lacks a feature providing controlled contraction of a helical form defined by distal portion (224).

As shown in FIG. 7, catheter body (230) of the present example includes a first layer (234), a second layer (232) disposed about first layer (234), and a third layer (238) disposed about a flex circuit substrate (302) and second layer (232). Lumen (236) is defined by first layer (234). First layer (234) of the present example comprises a braided construction formed of fibers or wires (e.g., non-ferromagnetic material, etc.), though first layer (234) may alternatively have any other suitable composition and/or configuration. In some versions, the inner sidewall of first layer (234) (i.e., the sidewall of lumen (236)) includes a lubricious coating or otherwise comprises a low friction material. This may promote sliding of guidewire (250) through catheter body (230).

Second layer (232) of the present example comprises a polymeric material that is bonded about first layer (234). By way of example only, second layer (232) may comprise a thermoplastic polyurethane (e.g., poly(etherurethane), etc.) and/or any other suitable material(s). In some variations, second layer (232) is omitted. For instance, some versions may omit second layer (232) when first layer (234) has sufficient thickness or otherwise has sufficient structural integrity (e.g., due to material properties, etc.) in the absence of second layer (232). In some versions, flex circuit substrate (302) effectively serves as second layer (232).

While tip member (226) of the present example comprises a separate component that is secured to the distal end of distal portion (224) of catheter body (230), some other versions may include tip member (226) as an integrally formed feature of catheter body (230). For instance, tip member (226) may comprise a distal extension of first layer (234), with or without second layer (232) further extending into this distal extension of first layer (234) to form tip member (226). In such versions, tip member (226) may (or may not) be formed into the same tapered shape as is shown in FIG. 6.

In the present example, flex circuit substrate (302) effectively extends about second layer (232) to an angular extent of approximately 270 degrees about a central longitudinal axis. Alternatively, flex circuit substrate (302) may effectively extend about second layer (232) to any other suitable angular extent about a central longitudinal axis. For instance, in some variations, flex circuit substrate (302) may wrap fully around second layer (232); and flex circuit substrate (302) may may even overlap itself due to the angular extent of wrapping. It should also be understood that different regions of flex circuit substrate (302) may extend to different respective angular extents at different longitudinal positions along the length of distal portion (224) of shaft assembly (220). In the present example, third layer (238) is applied about the exterior of flex circuit substrate (302), and the region of second layer (232) left exposed by flex circuit substrate (302), through a reflow process. By way of further example only, third layer (238) may comprise thermoplastic polyurethane (e.g., poly(etherurethane), etc.), silicone, and/or any other suitable material(s) that may be heated to provide the desired performance during a reflow process. Alternatively, third layer (238) may comprise any suitable material(s) and may be applied in any other suitable fashion. In the present example, layers (232, 238) have substantially strong bonding properties with first layer (234), with each other, and with flex circuit substrate (302). In some cases, such bonding may be achieved through the use of adhesive(s). In some other cases, such bonding is achieved through the application of heat and pressure (e.g., hot air reflow, etc.) and/or molding. Alternatively, any other suitable kind(s) of bonding may be used.

While third layer (238) and second layer (232) are depicted as being different materials in FIG. 7, third layer (238) and second layer (232) may comprise the same material in some versions. In other words, layers (232, 238) may effectively comprise a single layer with flex circuit substrate (302) embedded within that single layer.

As shown in FIGS. 2-4 and 6, tip member (226) of the present example includes a lumen (227) and an open distal end (228). By way of example only, lumen (227) and/or the opening at distal end (228) may have a diameter of approximately 0.3 mm or any other suitable diameter. Lumen (227) of tip member (226) is aligned with lumen (236) of catheter body (230), allowing guidewire (250) to pass through catheter body (230) and tip member (226). Lumens (236, 227) may also allow fluids (e.g., irrigation fluid such as saline, ethanol, contrast medium, etc.) to be expelled out through open distal end (228). Tip member (226) has a tapered configuration in this example, though tip member (226) may alternatively have any other suitable configuration. Like catheter body (230), tip member (226) is flexible. In the present example, tip member (226) is more flexible than catheter body (230). Increased flexibility in tip member (226) may facilitate navigation of shaft assembly (220) into tortuous, narrow anatomical passageways. In some versions, tip member (226) is more flexible than catheter body (230) because tip member (226) lacks a braided core like first layer (234) of catheter body (230). In addition, or in the alternative, tip member (226) may comprise a material that is more flexible than material(s) used to form second layer (232) and/or third layer (238) of catheter body (230). By way of example only, tip member (226) may comprise polyether block amide (e.g., PEBAX by Arkema of Colombes, France), thermoplastic polyurethane (e.g., TECOTHANE by Lubrizol Advanced Materials, Inc. of Cleveland, Ohio), silicone, or some other polymer, including combinations thereof. Alternatively, tip member (226) may comprise any other suitable material(s).

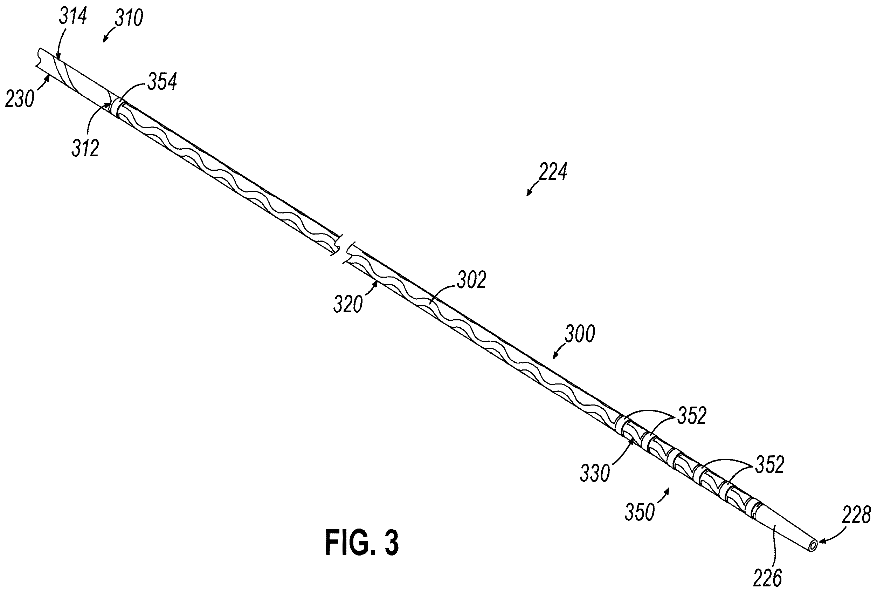

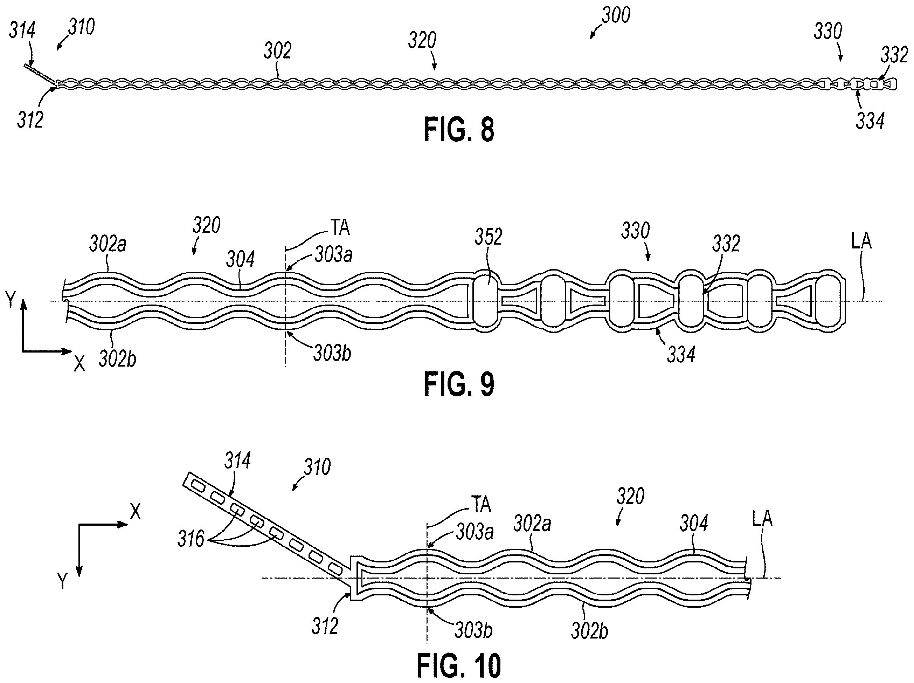

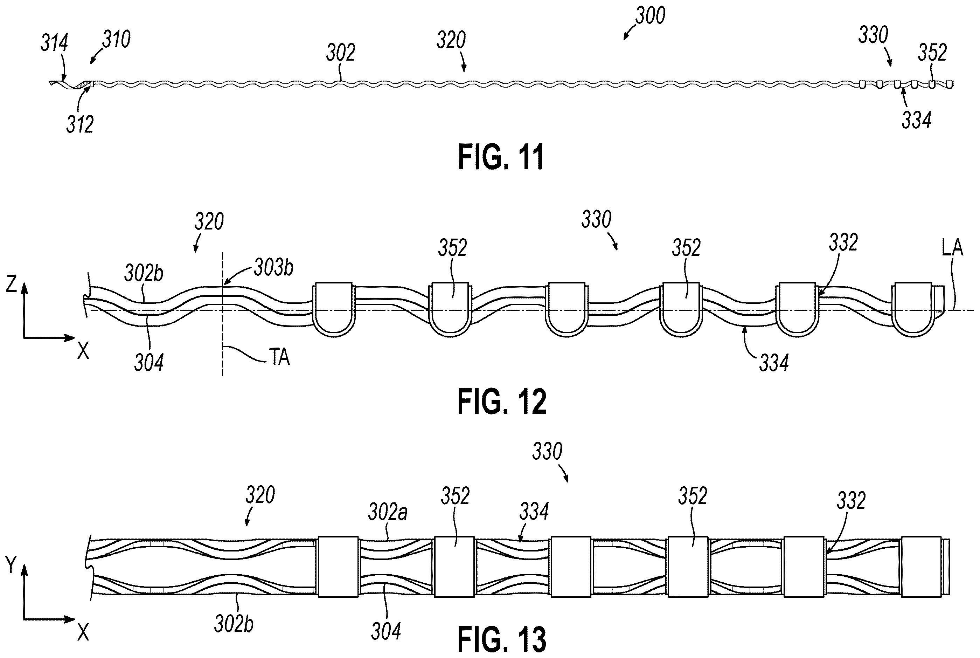

A flex circuit (300) is positioned along distal portion (224) of shaft assembly (220) and forms part of end effector (350) while also extending proximally relative to end effector (350). As shown in FIGS. 3-6, flex circuit (300) of the present example has a proximal portion (310), an intermediate portion (320), and a distal portion (330). FIGS. 8-10 show flex circuit (300) in a flat configuration, which is a configuration flex circuit (300) may have before flex circuit (300) is secured to catheter body (230). FIGS. 11-15 show flex circuit (300) in a wrapped configuration, which is a configuration flex circuit (300) may have after flex circuit (300) is secured to catheter body (230).

Flex circuit (300) comprises flexible substrate (302), traces (304), and electrodes (352). It should be understood that flex circuit (300) may have substantially more traces (304) than what is shown in the present drawings, as the number of depicted traces (304) is minimized in the present drawings for the sake of clarity. As used herein, the singular term "trace" shall be understood to include the plural term "traces." In other words, the depiction of a single trace (304) in the present drawings, and the description of "trace (304)" in the present specification, is contemplated to include scenarios where more than one trace is provided. The present disclosure should therefore not be read as contemplating that only a single trace would be provided in each instance where the drawings depict a single trace (304) and the specification refers to a "trace." The term "trace," and the corresponding depiction of a single trace, is just being used as a shorthand reference to one or more traces. The claims should also be understood such that the term "trace" may include "traces."

Flexible substrate (302) may comprise polyimide and/or any other suitable material(s). Traces (304) may comprise any suitable electrically conductive material. Traces (304) may be positioned on either surface or both surfaces of flexible substrate (302). At least some of traces (304) may be covered by third layer (238) of catheter body (230) as described above. Traces (304) may also be covered by another electrically insulating layer of plastic (e.g., polyimide, etc.) before third layer (238), as part of the manufacturing process of flex circuit (300), before third layer (238) is applied to flex circuit (300). In some such versions, the layers of flex circuit (300) may include a polyimide cover film, an adhesive, a conductive trace (e.g., trace (304)), a polyimide substrate (e.g., flexible substrate (302)), a conductive trace (e.g., trace (304)), an adhesive, and a polyimide cover film. Traces (304) may thus be provided on both sides of flexible substrate (302), with adhesive and cover film layers also being provided along both layers of traces (304) and both sides of flexible substrate (302) along regions left exposed by traces (304).

Electrodes (352) may be left uncovered by third layer (238) of catheter body (230), such that electrodes (352) may directly contact tissue, electrically conductive fluids, etc. Electrodes (352) may comprise an electrically conductive material such that each electrode (352) is operable to apply electrical energy to tissue as described above and as described in various references cited herein. By way of example only, each electrode (352) may comprise a noble metal, such as gold, platinum, ruthenium, rhodium, palladium, silver, osmium, iridium, etc. Alternatively, any other suitable electrically conductive material(s) may be used. In some versions, each electrode (352) is also operable to pick up electrical potentials in tissue (e.g., as part of an EP mapping process), as also described above and as described in various references cited herein.

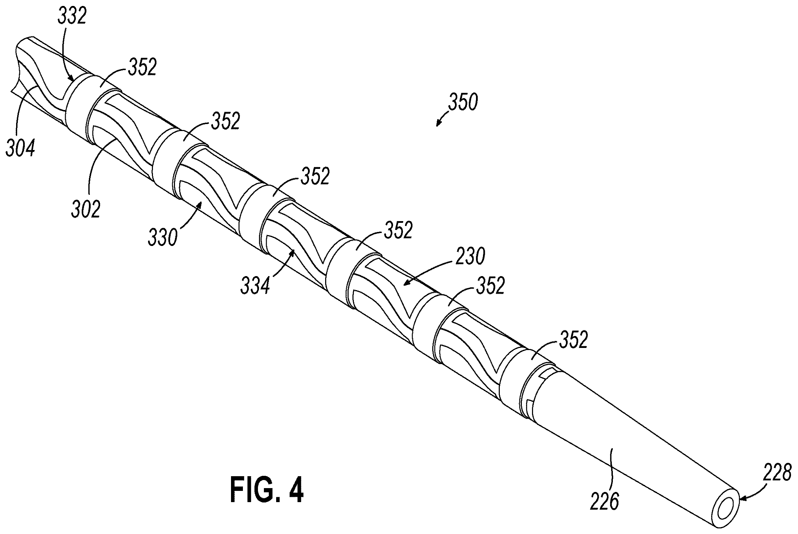

As also shown in FIGS. 3-6 and 8-15, the structural configuration of flex circuit (300) is different among proximal portion (310), intermediate portion (320), and distal portion (330). As best seen in FIGS. 4, 6, 9, and 12-13, flexible substrate (302) in distal portion (330) includes circumferentially extending portions (332) and longitudinally extending portions (334), with circumferentially extending portions (332) being longitudinally spaced apart from each other by respective pairs of longitudinally extending portions (334). Each circumferentially extending portion (332) has a respective electrode (352) applied thereto. Traces (304) extend along longitudinally extending portions (334). Portions of traces (304) may also extend along circumferentially extending portions (332). While flex circuit (300) of the present example has six circumferentially extending portions (332) and respective electrodes (352), other variations may have any other suitable number of circumferentially extending portions (332) and respective electrodes (352). By way of example only, some variations may have a number of electrodes (352) ranging from two to twenty; or any other suitable number of electrodes (352).

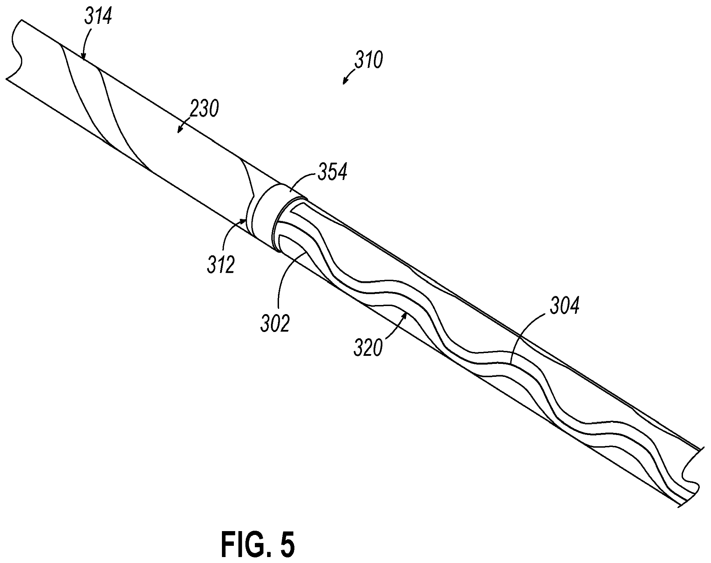

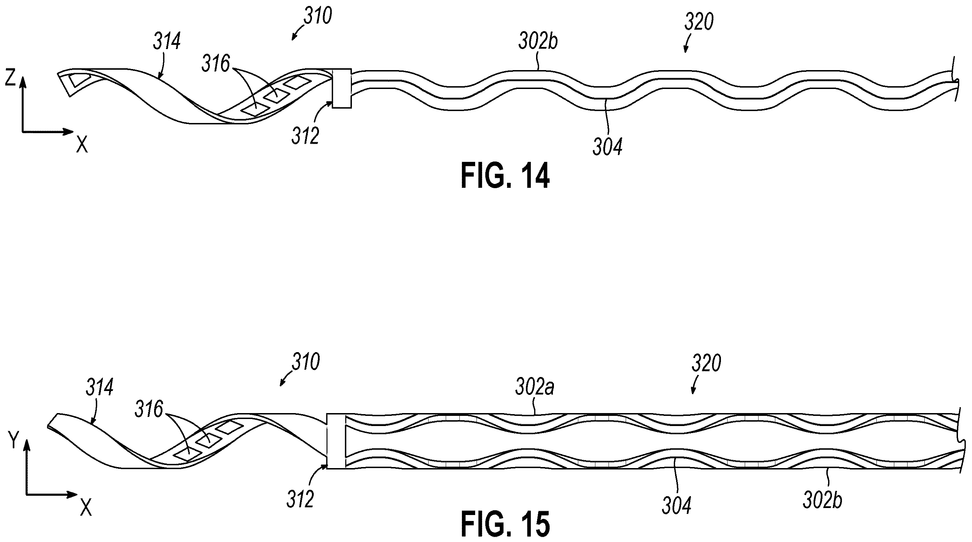

As best seen in FIGS. 3, 5, and 8-15, flexible substrate (302) in proximal portion (320) has a circumferentially extending portion (312), a tail portion (314), and a plurality of contact pads (316). Circumferentially extending portion (312) may extend about catheter body (230) along an angular extent of approximately 270 degrees. Alternatively, circumferentially extending portion (312) may extend about catheter body (230) along any other suitable angular extent. In the arrangement shown in FIGS. 3 and 5, an electrode (354) is positioned about circumferentially extending portion (312). In some versions, electrode (354) is used as a reference electrode. In some such versions, electrode (354) is used during an EP mapping procedure to pick up reference potentials from blood or saline that passes along end effector (350). Such reference potentials may be used to reduce noise or far field signals, as is known in the art. In addition, or in the alternative, electrode (354) may be used as an active or return pole during an ablation procedure, such as RF ablation or PFA, etc. As yet another variation, electrode (354) may be omitted, as is shown in FIGS. 14-15.

As best seen in FIGS. 8-10, when flex circuit (300) is in the flat configuration, tail portion (314) extends obliquely outwardly relative to a central longitudinal axis (LA). As best seen in FIGS. 11-15, when flex circuit (300) is in the wrapped configuration, tail portion (314) wraps about catheter body (230) in a helical configuration. The underside of tail portion (314) includes contact pads (316) in this example. Contact pads (316) are in electrical communication with traces (304) and electrodes (352). Contact pads (316) are configured to couple with wires, traces of another flex circuit, and/or other electrically conductive features on or in rigid shaft (223). In some versions of tail portion (314) may be positioned inside rigid shaft (223), such that contact pads (316) couple with wires, traces of another flex circuit, and/or other electrically conductive features that are inside rigid shaft (223). Such wires, flex circuits, and/or other electrically conductive features on or in rigid shaft (223) may be further electrically coupled with cable (216). Tail portion (314) may be incorporated into shaft (230) via third layer (238), which may be disposed about the outer surface of tail portion (314) along the entire length of tail portion (314). In some other versions, tail portion (314) extends into the interior of rigid shaft (223) and may extend into handle assembly (210). It should also be understood that contact pads (316) may be positioned on top of tail portion (314), in addition to or as an alternative to contact pads (316) being positioned on the underside of tail portion (314). Alternatively, flex circuit (300) may be electrically coupled with features of cable (216) in any other suitable fashion.

As best seen in FIGS. 3, 5, and 8-15, flexible substrate (302) in intermediate portion (320) extends longitudinally and has two legs (302a, 302b) defining an undulating or serpentine configuration. As best seen in FIGS. 8-10, when flex circuit (300) is in the flat configuration, a first serpentine leg (302a) of flexible substrate (302) is positioned on a first side of a central longitudinal axis (LA) (which is oriented along the x-dimension in FIGS. 8-10) while second serpentine leg (302b) of flexible substrate (302) is positioned on a second side of a central longitudinal axis (LA). Serpentine legs (302a, 302b) are spaced apart from each other along the y-dimension. Serpentine legs (302a, 302b) are positioned relative to each other along the x-dimension such that respective peaks (303a, 303b) of serpentine legs (302a, 302b) are aligned with each other along a transverse axis (TA) (which is oriented along the y-dimension in FIGS. 8-10). In other words, if serpentine legs (302a, 302b) were viewed as respective waveforms plotted along the x-y plane in FIGS. 8-10, these waveforms would be out of phase with each other by 180 degrees when flex circuit (300) is in the flat configuration.

As best seen in FIGS. 11-15, when flex circuit (300) is in the wrapped configuration, serpentine legs (302a, 302b) are positioned relative to each other along the x-dimension such that respective peaks (303a, 303b) of serpentine legs (302a, 302b) are aligned with each other along another transverse axis (TA) (which is oriented along the z-dimension in FIGS. 11-15). In other words, if serpentine legs (302a, 302b) were viewed as respective waveforms plotted along the x-z plane in FIGS. 11-12 and 14, these waveforms would be in phase with each when flex circuit (300) is in the wrapped configuration.

In the present example, trace (304) (which may in fact include more than one trace, as noted above) extends along the entire length of the serpentine configuration of flexible substrate (302) throughout at least intermediate portion (320). This trace (304) continues along both serpentine legs (302a, 302b) and connects between serpentine legs (302a, 302b). For instance, trace may span across a circumferentially extending portion (332) of distal portion (330) of flex circuit (300) to thereby connect between serpentine legs (302a, 302b). Trace (304) may also span across circumferentially extending portion (312) of proximal portion (320) of flex circuit (300) to thereby connect between serpentine legs (302a, 302b). By extending along both serpentine legs (302a, 302b) and a circumferentially extending portion (332), trace (304) forms a loop configuration. In cases where trace (304) also spans across circumferentially extending portion (312) of proximal portion (320) of flex circuit (300) to thereby connect between serpentine legs (302a, 302b), trace (304) may form a multi-wrap coil loop configuration.

In some versions, the loop formed by trace (304) only extends along the proximal-most circumferentially extending portion (332) of distal portion (330) of flex circuit (300), such that the loop does not extend along the length of distal portion (330) of flex circuit and only extends along the length of intermediate portion (320) of flex circuit (300). In some other versions, the loop formed by trace (304) extends along the distal-most circumferentially extending portion (332) of distal portion (330) of flex circuit (300), such that the loop extends along the length of distal portion (330) of flex circuit in addition to the length of intermediate portion (320) of flex circuit (300).

In the present example the loop formed by trace (304) along intermediate portion (320) (and, in some cases, also along distal portion (330)) provides a position sensor as described above. In other words, the loop formed by trace (304) along intermediate portion (320) (and, in some cases, also along distal portion (330)) acts as an inductive coil that provides electrical signals in response to the presence of an alternating electromagnetic field generated by field generators (20). These electrical signals may be indicative of the of the real-time position and orientation of end effector (350) within the patient (PA). The processor of console (12) may receive these position indicative signals via cable (216) and process the position indicative signals to thereby determine the real-time position and orientation of end effector (350) within the patient (PA). The processor of console (12) may thus drive display (18) to indicate the real-time position and orientation of end effector (350) within the patient (PA) in relation to preoperative images of patient anatomy, in relation to digital models of patient anatomy, etc., as described above and as described in various references cited herein.

In some variations, legs (302a, 302b) of flexible substrate (302) along opposite sides of the central longitudinal axis (LA) are straight (e.g., just extending longitudinally) rather than having a serpentine configuration. In such variations, the loop formed by trace (304) may still effectively provide a position sensor. With legs (302a, 302b) of flexible substrate (302) along opposite sides of the central longitudinal axis (LA) having a serpentine configuration in the present example, the loop formed by trace (304) also effectively provides a position sensor, but the serpentine configuration of legs (302a, 302b) of flexible substrate (302) may further facilitate flexing of flex circuit (300) along intermediate portion (320). In some cases, the serpentine configuration of legs (302a, 302b) of flexible substrate (302) does not affect the performance of the loop formed by trace (304) as a position sensor, as compared to the performance of the loop formed by trace (304) as a position sensor in versions where legs (302a, 302b) are straight. Also in some cases, the serpentine configuration of legs (302a, 302b) of flexible substrate (302) does not create or enhance a flexing bias in distal portion (224) of shaft assembly (220). In other words, the serpentine configuration of legs (302a, 302b) of flexible substrate (302) does not provide a resilient bias urging distal portion (224) of shaft assembly (220) toward a straight configuration along the central longitudinal axis (LA).

To the extent that some conventional catheter instruments include position sensors at an end effector or otherwise at the distal tip of a shaft assembly, catheter assembly (200) of the present example may substantially differ from such conventional catheter instruments in that the position sensor provided by the loop formed by trace (304) along intermediate portion (320) (and, in some cases, also along distal portion (330)) extends along a substantial portion of the overall length of shaft assembly (220). For instance, the position sensor provided by the loop formed by trace (304) may have a length of at least approximately 5 cm, of at least approximately 10 cm, ranging from approximately 5 cm to approximately 20 cm, ranging from approximately 10 cm to approximately 15 cm, or of approximately 13 cm. Alternatively, the position sensor provided by the loop formed by trace (304) may have any other suitable length.

In relation to the overall length of shaft assembly (220) the position sensor provided by the loop formed by trace (304) may extend along at least approximately 2.5% of the overall length of shaft assembly (220), along at least approximately 3% of the overall length of shaft assembly (220), along at least approximately 5% of the overall length of shaft assembly (220), along at least approximately 6.5% of the overall length of shaft assembly (220), along at least approximately 7.5% of the overall length of shaft assembly (220), along at least approximately 8.5% of the overall length of shaft assembly (220), along at least approximately 10% of the overall length of shaft assembly (220), along at least approximately 11.5% of the overall length of shaft assembly (220), along at least approximately 13% of the overall length of shaft assembly (220), along at least approximately 15% of the overall length of shaft assembly (220), or along at least approximately 20% of the overall length of shaft assembly (220). Alternatively, the length of the position sensor provided by the loop formed by trace (304) may have any other suitable relationship with the overall length of shaft assembly (220).

In relation to the outer diameter of shaft assembly (220), the length of the position sensor provided by the loop formed by trace (304) may be greater than approximately 10 times the outer diameter of shaft assembly (220), greater than approximately 20 times the outer diameter of shaft assembly (220), greater than approximately 25 times the outer diameter of shaft assembly (220), greater than approximately 50 times the outer diameter of shaft assembly (220), greater than approximately 75 times the outer diameter of shaft assembly (220), greater than approximately 100 times the outer diameter of shaft assembly (220), greater than approximately 125 times the outer diameter of shaft assembly (220), greater than approximately 145 times the outer diameter of shaft assembly (220), greater than approximately 200 times the outer diameter of shaft assembly (220), greater than approximately 250 times the outer diameter of shaft assembly (220), or greater than approximately 300 times the outer diameter of shaft assembly (220). Alternatively, the length of the position sensor provided by the loop formed by trace (304) may have any other suitable relationship with the outer diameter of shaft assembly (220).

Regardless of whether the length of the position sensor provided by the loop formed by trace (304) is compared against the overall length of shaft assembly (220) or the outer diameter of shaft assembly (220), the relative length of the position sensor provided by the loop formed by trace (304) may be considered substantial, particularly in comparison to similar relative lengths provided by conventional position sensors. This substantial relative length of the position sensor may be counterintuitive, as conventional instrument design considerations may tend to focus on minimization of component size. In other words, conventional position sensors may tend to be as compact as possible, such that the position sensor provided by the loop formed by trace (304) may be substantially larger than conventional position sensors. Similarly, the surface area bounded by trace (304) may be substantially larger than the surface area bounded by conventional position sensors. Conventional position sensor design may also indicate that position sensors should not flex during use; whereas the position sensor provided by the loop formed by trace (304) may tend to flex substantially during use; and this flexing may be even more likely due to the substantial length of the position sensor provided by the loop formed by trace (304). In other words, the substantial length of the position sensor provided by the loop formed by trace (304) may promote flexing of the position sensor during use.

Another way in that catheter assembly (200) of the present example may substantially differ from conventional catheter instruments with position sensors in that the position sensor provided by the loop formed by trace (304) may tend to substantially flex during operation of catheter assembly (200). This flexing may be facilitated by the serpentine configuration of flexible substrate (302) along intermediate portion (320) of flex circuit (300). This flexing of the position sensor may be due in part to the substantial length of the position sensor in the present example. By contrast, conventional position sensors may be substantially shorter and/or may be longitudinally constrained to components of catheter assemblies that do not flex. In other words, conventional position sensors may tend to either not flex at all during use or may only flex minimally during use, such that conventional position sensors would not flex nearly as substantially as the position sensor provided by the loop formed by trace (304).

### III. Examples of Elongate Position Sensor Arrangements

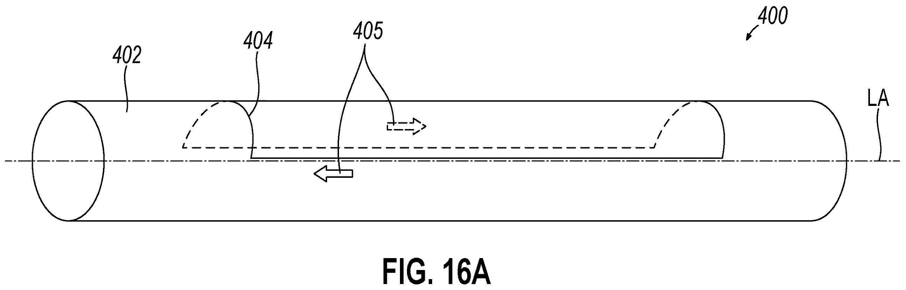

As noted above, the loop formed by trace (304) along intermediate portion (320) (and, in some cases, also along distal portion (330)) may provide a position sensor that provides position indicative signals in the presence of an alternating electromagnetic field. As also noted above, the loop formed by trace (304) may be considered as forming a generally rectangular shape draped about catheter body (230) extending longitudinally along part of the length of catheter body (230). This arrangement is shown schematically in FIG. 16A. Specifically, FIG. 16A shows a first example of an elongate position sensor arrangement (400) where a loop (404) is draped over an upper portion of a catheter body (402). Loop (404) thus schematically represents the loop formed by trace (304) in flex circuit (300). The length of the rectangle defined by loop (404) is driven by the length of the flex circuit in which loop (404) is incorporated; and the width of the rectangle defined by loop is driven by the diameter of catheter body (402).

Arrows (405) indicate the direction of an electrical current that may be generated in in loop (404) by an alternating electromagnetic field (e.g., as generated by field generators (20)). The direction indicated by arrows (405) in FIG. 16A is just illustrative, and the electrical current may in fact flow in the opposite direction depending on the field orientation. In the example shown in FIG. 16A, the circumferentially extending segments of loop (404) each have an angular extent of approximately 180 degrees about the central longitudinal axis (LA) of catheter body (402), such that the longitudinally extending segments of loop (404) are angularly offset from each other by approximately 180 degrees about the central longitudinal axis (LA) of catheter body (402). This relative positioning of the longitudinally extending segments of loop (404) may tend to maximize the sensitivity of the position sensor provided by loop (404). Alternatively, the circumferentially extending segments of loop (404) may have any other suitable angular extent; and the longitudinally extending segments of loop (404) may be angularly offset from each other by any other suitable angular distance.

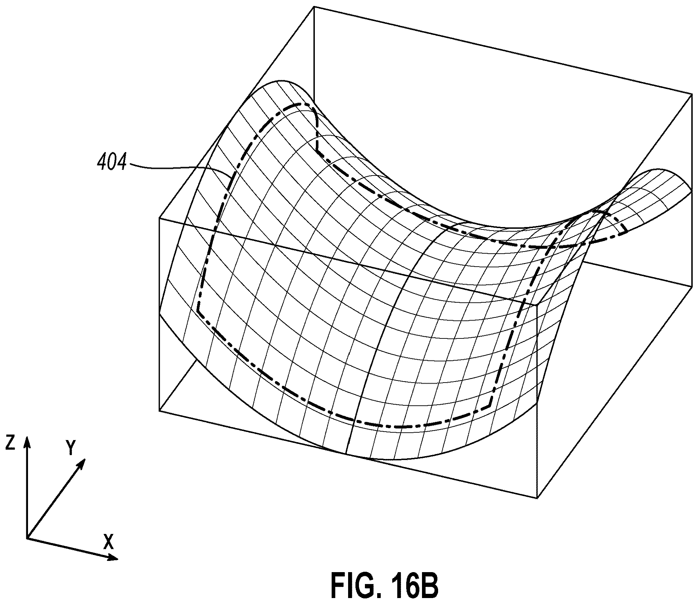

In the state of operation shown in FIG. 16A, catheter body (402) is in a non-bent state, such that loop (404) is in a non-bent state. During normal operation, catheter body (402) may bend in numerous different directions as catheter body (402) traverses tortuous anatomical passageways as described herein. Loop (404) will bend with catheter body (402). FIG. 16B shows an example of loop (404) in such a bent state. As shown, loop (404) is bent along an x-z plane in this example, while loop (404) extends longitudinally along an x-direction. As also shown, loop (404) defines a saddle-shaped plane, or extends along a saddle-shaped plane, when loop (404) is bent in this fashion. In some other scenarios (e.g., when catheter body (402) and loop (404) are bent along the x-y plane), loop (404) may define a different shape when loop (404) is in a bent state.

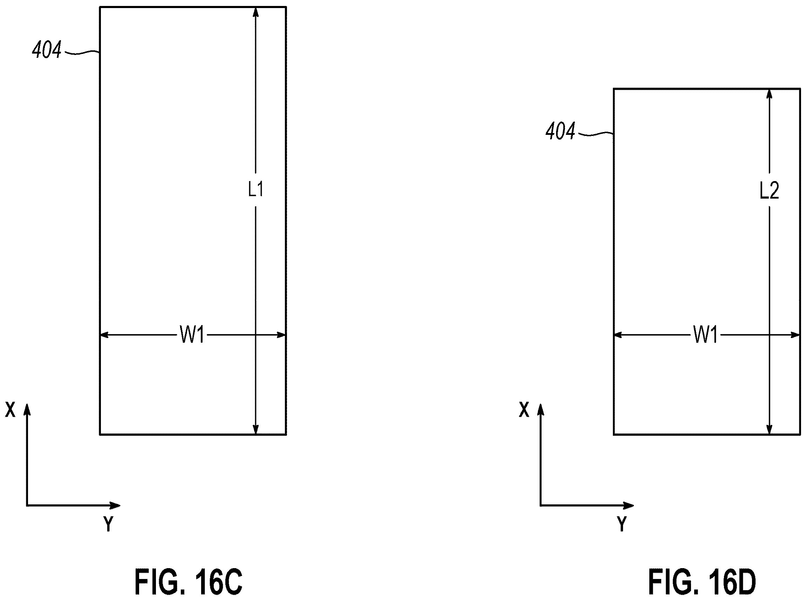

As schematically shown in FIG. 16C, loop (404) may have a first effective length (L1) and a first effective width (W1) when loop (404) is in a non-bent state (e.g., the state shown in FIG. 16A). In this non-bent state, loop (404) may effectively bound a first area (i.e., L1 x W1) along a certain two-dimensional plane (e.g., the x-y plane as shown in FIG. 16C). As schematically shown in FIG. 16D, loop (404) may have a second effective length (L2), while maintaining the first effective width (W1), when loop (404) is in a bent state (e.g., the state shown in FIG. 16B). In this bent state, loop (404) may effectively bound a second area (i.e., L2 x W1) along the same two-dimensional plane (e.g., the x-y plane as shown in FIG. 16D). In this example, the second effective length (L2) is smaller than the first effective length (L1). Consequently, the second area (i.e., L2 x W1) along the two-dimensional plane is smaller than the first area (i.e., L1 x W1) along the two-dimensional plane. In other words, the area defined by the effective length (L) and the effective width (W) of loop (404) along a two-dimensional reference plane may tend to get smaller when catheter body (402) and loop (404) are bent, particularly when catheter body (402) and loop (404) are bent.

In some other scenarios, the effective width (W) of loop (404) may change when catheter body (402) and loop (404) are bent, in addition to or in lieu of the effective length (L) of loop (404) changing when catheter body (402) and loop (404) are bent. This may depend on the direction in which catheter body (402) and loop (404) are bent. In any of these cases, the area defined by the effective length and width of loop (404) along a given two-dimensional plane may change when catheter body (402) and loop (404) are bent, such as by getting smaller when catheter body (402) and loop (404) are bent. In some versions, catheter assembly (200) is constructed such that the area defined by the effective length (L) and the effective width (W) of loop (404) along a two-dimensional reference plane (including when catheter body (402) and loop (404) are in a bent state) is at least approximately 150 mm²; at least approximately 175 mm²; at least approximately 200 mm²; at least approximately 225 mm²; at least approximately 250 mm²; at least approximately 275 mm²; or at least approximately 300 mm². Alternatively, loop (404) may define any other suitable area along a two-dimensional reference plane when loop (404) is in a non-bent state or in a bent state.

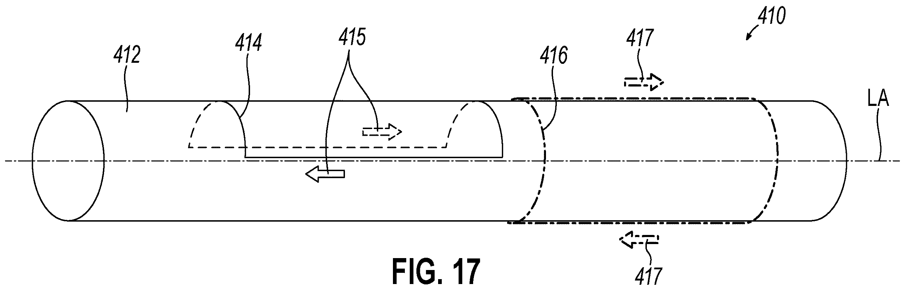

FIG. 17 shows a second example of an elongate position sensor arrangement (410). In this arrangement (410), a first loop (414) is draped over an upper portion of a catheter body (412), at a first angular position and at a first longitudinal position; and a second loop (416) is draped over a side portion of catheter body (412), at a second angular position and at a second longitudinal position. Loops (414, 416) are electrically isolated relative to each other in this example. The second angular position is angularly offset from the first angular position by approximately 90 degrees about the central longitudinal axis (LA) of catheter body (412). The second longitudinal position is distal to the first longitudinal position. The arrangement of loops (414, 416) is such that no portion of loop (414) overlaps with any portion of loop (416). This longitudinal spacing of loops (414, 416) may maximize flexibility of arrangement (410).

Arrows (415) indicate the direction of an electrical current that may be generated in in loop (414) by an alternating electromagnetic field (e.g., as generated by field generators (20)). In the example shown in FIG. 17, the circumferentially extending segments of loop (414) each have an angular extent of approximately 180 degrees about the central longitudinal axis (LA) of catheter body (412), such that the longitudinally extending segments of loop (414) are angularly offset from each other by approximately 180 degrees about the central longitudinal axis (LA) of catheter body (412). This relative positioning of the longitudinally extending segments of loop (414) may tend to maximize the sensitivity of the position sensor provided by loop (414). Alternatively, the circumferentially extending segments of loop (414) may have any other suitable angular extent; and the longitudinally extending segments of loop (414) may be angularly offset from each other by any other suitable angular distance.

Arrows (417) indicate the direction of an electrical current that may be generated in in loop (416) by an alternating electromagnetic field (e.g., as generated by field generators (20)). In the example shown in FIG. 17, the circumferentially extending segments of loop (416) each have an angular extent of approximately 180 degrees about the central longitudinal axis (LA) of catheter body (412), such that the longitudinally extending segments of loop (416) are angularly offset from each other by approximately 180 degrees about the central longitudinal axis (LA) of catheter body (412). This relative positioning of the longitudinally extending segments of loop (416) may tend to maximize the sensitivity of the position sensor provided by loop (416). Alternatively, the circumferentially extending segments of loop (416) may have any other suitable angular extent; and the longitudinally extending segments of loop (416) may be angularly offset from each other by any other suitable angular distance.

The presence of two loops (414, 416), and the angular offset of approximately 90 degrees between loops (414, 416), may provide arrangement (410) with position sensitivity in two degrees of freedom, which may thus provide enhanced real-time position information as compared to the real-time position information provided by arrangement (400). While only two loops (414, 416) are provided in arrangement (410), variations of arrangement (410) may include three or more loops, with each loop providing its own respective position indicative signal.

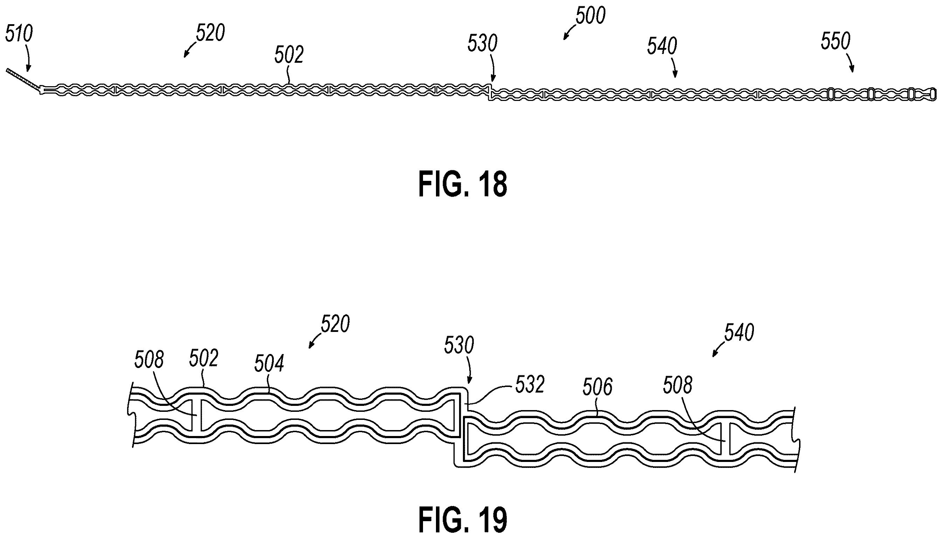

FIGS. 18-19 show an example of a flex circuit (500) that may be used to provide arrangement (410) of FIG. 17. Flex circuit (500) is shown in a flat configuration but it should be understood that flex circuit (500) may be wrapped about a catheter body as described above in the context of flex circuit (300). Flex circuit (500) of this example comprises a flexible substrate (502) having a proximal portion (510), a first intermediate portion (520), a transition portion (530), a second intermediate portion (540), and a distal portion (550). Proximal portion (510) of flex circuit (500) may be configured and operable like proximal portion (310) of flex circuit (300) described above. Similarly, distal portion (550) of flex circuit (500) may be configured and operable like distal portion (330) of flex circuit (300) described above.

Like intermediate portion (320) of flex circuit (300), first intermediate portion (520) of flex circuit (500) has two legs, each defining an undulating or serpentine configuration. Also like intermediate portion (320) of flex circuit (300), second intermediate portion (540) of flex circuit (500) has two legs, each defining an undulating or serpentine configuration. Unlike intermediate portion (320) of flex circuit (300), each intermediate portion (520, 540) of flex circuit (500) includes a plurality of transversely oriented webs (508) joining the serpentine legs of flexible substrate (502). Webs (508) are longitudinally spaced apart from each other along the length of each intermediate portion (520, 540). Webs (508) may prevent serpentine legs of flexible substrate (502) from further moving apart from each other along a transverse axis, such that webs (508) may provide consistent spacing between the serpentine legs of flexible substrate (502) along the length of each intermediate portion (520, 540). In the present example, there are no electrical traces spanning across webs (508), though other variations may provide electrical traces spanning across webs (508).

Transition portion (530) of flexible substrate (502) joins intermediate portions (520, 540) together. In the flat configuration shown in FIGS. 18-19, transition portion (530) is oriented transversely; though in a wrapped configuration (when flex circuit (500) is wrapped about a catheter body), transition portion extends along a circumferential path.

First intermediate portion (520) includes a trace (504) that extends along both serpentine legs of flexible substrate (502) along the full length of first intermediate portion (520). Trace (504) also spans across transition portion (530). Trace (504) thus forms a loop providing a position sensor, as represented schematically by first loop (414) in position sensor arrangement of FIG. 17. Second intermediate portion (540) includes a trace (506) that extends along both serpentine legs of flexible substrate (502) along the full length of second intermediate portion (540). Trace (506) also spans across transition portion (530). Trace (506) thus forms a loop providing a position sensor, as represented schematically by second loop (416) in position sensor arrangement of FIG. 17. In the present example, traces (504, 506) are electrically isolated relative to each other. It should nevertheless be understood that a portion of trace (506) may extend along first intermediate portion (520) to reach a corresponding set of contact pads on proximal portion (510), to thereby permit communication of position indicative signals from the loop formed by trace (506) to a processor (e.g., the processor of console (12), etc.).

As described above with respect to loops (414, 416) of arrangement (410), intermediate portions (520, 540) are positioned relative to each other such that the loops formed by traces (504, 506) are angularly offset from each other by approximately 90 degrees about a central longitudinal axis of a catheter body when flex circuit (500) is wrapped about the catheter body. This offset is provided by the configuration of transition portion (530) in this example. In cases where three or more loops are provided, a flex circuit may include a modified version of transition portion (530) and/or more than one transition portion to accommodate any desired number of angular offsets corresponding to the number of loops.

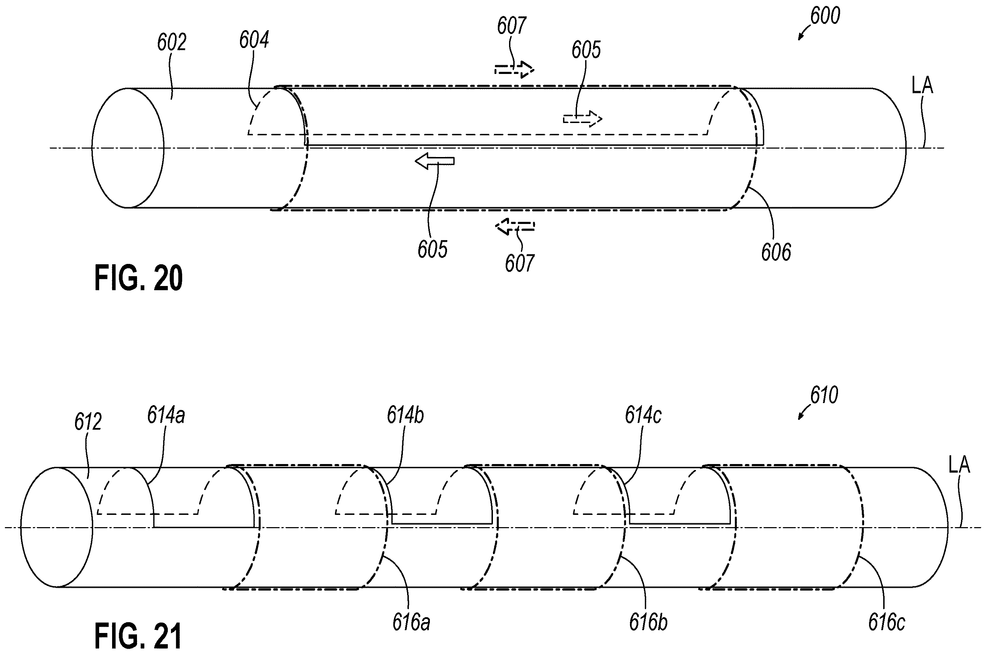

FIG. 20 shows a third example of an elongate position sensor arrangement (600). In this arrangement (600), a first loop (604) is draped over an upper portion of a catheter body (602), at a first angular position and at a first longitudinal position; and a second loop (606) is draped over the upper portion of catheter body (602), at a second angular position and at the first longitudinal position. Loops (604, 606) are electrically isolated relative to each other in this example. The second angular position is angularly offset from the first angular position by approximately 90 degrees about the central longitudinal axis (LA) of catheter body (602). Loops (604, 606) are longitudinally coextensive with each other in this example. The arrangement of loops (604, 606) is such that an angular segment of loop (604) overlaps with an angular segment of loop (606). In the present example, this angular overlap extends along approximately 90 degrees of the angular extent of each loop (604, 606). This relative spacing of loops (604, 606) may minimize the overall length of arrangement (600).

Arrows (605) indicate the direction of an electrical current that may be generated in in loop (604) by an alternating electromagnetic field (e.g., as generated by field generators (20)). In the example shown in FIG. 20, the circumferentially extending segments of loop (604) each have an angular extent of approximately 180 degrees about the central longitudinal axis (LA) of catheter body (602), such that the longitudinally extending segments of loop (602) are angularly offset from each other by approximately 180 degrees about the central longitudinal axis (LA) of catheter body (602). This relative positioning of the longitudinally extending segments of loop (604) may tend to maximize the sensitivity of the position sensor provided by loop (604). Alternatively, the circumferentially extending segments of loop (604) may have any other suitable angular extent; and the longitudinally extending segments of loop (604) may be angularly offset from each other by any other suitable angular distance.

Arrows (607) indicate the direction of an electrical current that may be generated in in loop (606) by an alternating electromagnetic field (e.g., as generated by field generators (20)). In the example shown in FIG. 20, the circumferentially extending segments of loop (606) each have an angular extent of approximately 180 degrees about the central longitudinal axis (LA) of catheter body (602), such that the longitudinally extending segments of loop (606) are angularly offset from each other by approximately 180 degrees about the central longitudinal axis (LA) of catheter body (602). This relative positioning of the longitudinally extending segments of loop (606) may tend to maximize the sensitivity of the position sensor provided by loop (606). Alternatively, the circumferentially extending segments of loop (606) may have any other suitable angular extent; and the longitudinally extending segments of loop (606) may be angularly offset from each other by any other suitable angular distance.

The presence of two loops (604, 606), and the angular offset of approximately 90 degrees between loops (604, 606), may provide arrangement (600) with position sensitivity in two degrees of freedom, which may thus provide enhanced real-time position information as compared to the real-time position information provided by arrangement (400). While only two loops (604, 606) are provided in arrangement (600), variations of arrangement (600) may include three or more loops, with each loop providing its own respective position indicative signal.

FIG. 21 shows a fourth example of an elongate position sensor arrangement (610). In this arrangement (610), a set of first loop segments (614a, 614b, 614c) is draped over an upper portion of a catheter body (612), at a first angular position about a central longitudinal axis (LA); set of second loop segments (616a, 616b, 616c) is draped over side portion of a catheter body (612), at a second angular position about a central longitudinal axis (LA). The second angular position is angularly offset from the first angular position by approximately 90 degrees about the central longitudinal axis (LA) of catheter body (412). The combination of angular and longitudinal positioning of loop segments (614a, 614b, 614c) relative to loop segments (616a, 616b, 616c) is such that loop segments (614a, 614b, 614c) area interleaved with loop segments (616a, 616b, 616c). However, no portion of any loop segment (614a, 614b, 614c) from the first set overlaps with any portion of any loop segment (616a, 616b, 616c) of the second set.

In the present example, loop segments (614a, 614b, 614c) of the first set are joined together in series (e.g., by a shared set of traces). Similarly, loop segments (616a, 616b, 616c) of the second set are joined together in series (e.g., by a shared set of traces). Loop segments (614a, 614b, 614c) of the first set are electrically isolated relative to loop segments (616a, 616b, 616c) of the second set in this example. While FIG. 21 does not include arrows to indicate the direction of an electrical current that may be generated in in each loop segment (614a, 614b, 614c, 616a, 616b, 616c) by an alternating electromagnetic field (e.g., as generated by field generators (20)), such current may be generated in a manner similar to that described above with respect to arrangements (400, 410, 600).

With loop segments (614a, 614b, 614c) of the first set being joined together in series, the first set of loop segments (614a, 614b, 614c) may cooperate to effectively provide a first single loop and thereby provide real-time position indicative signals in a first degree of freedom. Similarly, with loop segments (616a, 616b, 616c) of the second set being joined together in series, the second set of loop segments (616a, 616b, 616c) may cooperate to effectively provide a second single loop and thereby provide real-time position indicative signals in a second degree of freedom. Arrangement (610) may thus provide enhanced real-time position information as compared to the real-time position information provided by arrangement (400). While only effective loops are provided by loop segments (614a, 614b, 614c, 616a, 616b, 616c) in arrangement (610), variations of arrangement (610) may include additional loop segments effectively providing three or more effective loops, with each effective loop providing its own respective position indicative signal.

### IV. Examples of Electrical Paths

As noted above, electrodes (352) of end effector (350) may be used in numerous ways. For instance, electrodes (352) may be used to provide EP mapping and/or other diagnostic functionality. In addition, or in the alternative, electrodes (352) may be used to provide ablation and/or other therapeutic functionality. In the context of ablation, electrodes (352) may be used to apply RF energy tissue to ablate the tissue. In addition, or in the alternative, electrodes (352) may be used to apply PFA or some other form of electrical energy to ablate the tissue. Regardless of whether electrodes (352) are used to provide RF ablation, PFA, or some other form of electrical ablation, there are numerous permutations in which one or more different electrodes (352) may be activated at different polarities to apply electrical energy to tissue. Various examples of ways in which electrodes (352) may be used to apply electrical energy to tissue, either through direct contact with tissue or through indirect contact with tissue, will be described in greater detail below.

### A. Example of Electrical Path Between Electrodes and Ground Pad

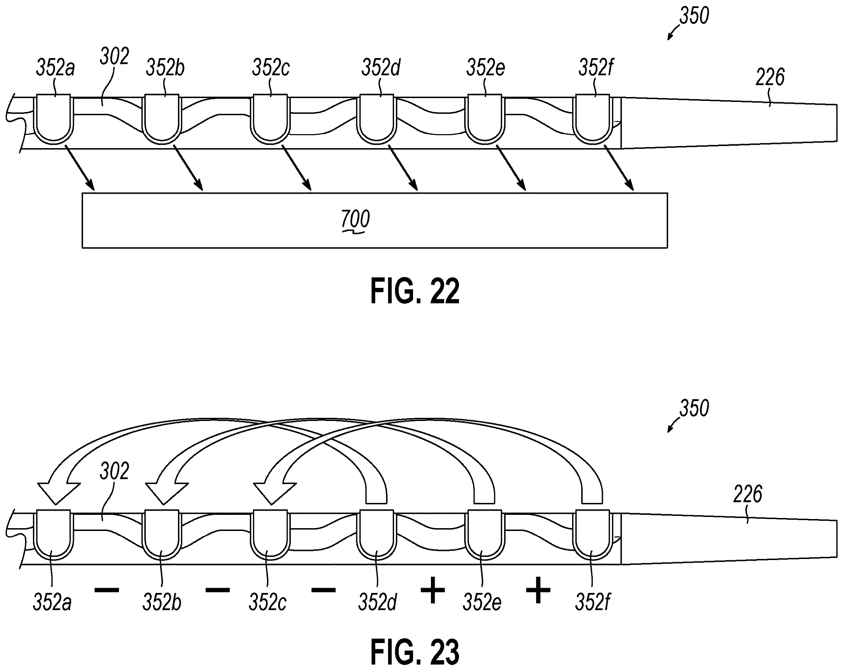

FIG. 22 shows electrodes (352) of end effector (350) being used with a ground pad (700) to provide a first example of an electrical path. In this example, end effector (350) is used as a monopolar instrument, such that one or more of electrodes (352) may be active while ground pad (700) provides a return path. Ground pad (700) may take any suitable form, including but not limited to a pad on which the patient (PA) rests, a patch adhered to the skin of the patient (PA), etc. When end effector (350) is positioned in the targeted anatomical space (e.g., the Vein of Marshall or other small vessel branching out from the coronary sinus), one or more of electrodes (352) may be placed in contact with tissue and then activated with monopolar electrical energy. The electrical energy may flow between the activated electrode(s) (352) and ground pad (700) via adjacent tissue, which may result in ablation of the tissue.

### B. Example of Electrical Path Between Combination of Electrodes

In some scenarios, it may be undesirable to use a ground pad (700), such that it may be more desirable to use electrodes (352) to apply bipolar electrical energy to tissue. This may allow the physician (PH) to ablate tissue in a more precise, targeted fashion. While bipolar electrical energy may be applied to tissue in versions of end effector (350) having only one electrode (352), versions of end effector (350) having three or more electrodes (352) may provide even greater control over formation of lesions and/or other ablation results by allowing the physician (PH) to make ad hoc discrete combinations of which electrodes (352) serve as active electrodes and which electrodes (352) serve as return electrodes at a given moment during an ablation procedure. Such control over bipolar assignments among electrodes (352) may allow the physician to fine tune the shape, gradient, and/or other characteristics of the lesion formed by the ablation process (and/or other results of the ablation process). This may in turn minimize the risk of undesirable gaps being left between or within ablated regions of tissue.

FIGS. 23-27 provide examples of different examples of which electrode(s) (352) may serve as active electrodes and which electrode(s) (352) may serve as return electrodes at a given moment during an ablation procedure. The examples shown in FIGS. 23-27 are just illustrative examples. Any one of electrodes (352), or group of two or more electrodes (352), may be used to establish an electrical bipole with any other electrode (352), or group of two or more electrodes (352), to deliver electrical energy. The following examples are not intended to be exhaustive.

FIG. 23 shows electrodes (352) of end effector (350) being used together to provide a second example of an electrical path. In this example, distal-most electrodes (352, 352e, 352f) serve as active electrodes while proximal-most electrodes (352a, 352b, 352c) serve as return electrodes. In some such versions, distal-most electrodes (352d, 352e, 352f) are activated simultaneously while proximal-most electrodes (352a, 352b, 352c) simultaneously serve as return electrodes. In some other versions, distal-most electrodes (352d, 352e, 352f) are activated in a sequence while proximal-most electrodes (352a, 352b, 352c) sequentially serve as return electrodes. In some such versions, electrode (352a) serves as a return electrode while electrode (352d) is activated, electrode (352b) serves as a return electrode while electrode (352e) is activated, and electrode (352c) serves as a return electrode while electrode (352f) is activated. Such a sequence may occur in any suitable order or arrangement. Moreover, bipolar sets may be formed between any suitable pair of electrodes (352a, 352b, 352c, 352d, 352e, 352f) at any moment in time.

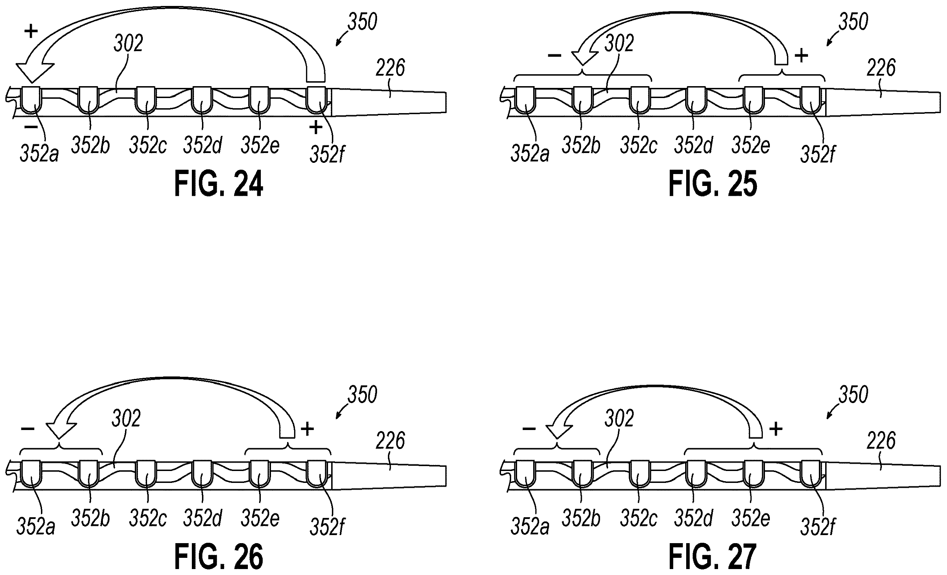

FIG. 24 shows electrodes (352) of end effector (350) being used together to provide a third example of an electrical path. In this example, distal-most electrode (352f) serves as an active electrode while proximal-most electrode (352a) serves as a return electrode.

FIG. 25 shows electrodes (352) of end effector (350) being used together to provide a fourth example of an electrical path. In this example, electrodes (352e, 352f) together/simultaneously serve as an active electrode while electrodes (352a, 352b, 352c) together/simultaneously serve as a return electrode. Thus, it is not necessary for there to be 1:1 parity between the number of electrodes (352) serving as an active electrode and the number of electrodes (352) serving as a return electrode at a given moment in time.

FIG. 26 shows electrodes (352) of end effector (350) being used together to provide a fifth example of an electrical path. In this example, electrodes (352e, 352f) together/simultaneously serve as an active electrode while electrodes (352a, 352b) together/simultaneously serve as a return electrode.

FIG. 27 shows electrodes (352) of end effector (350) being used together to provide a sixth example of an electrical path. In this example, electrodes (352d, 352e, 352f) together/simultaneously serve as an active electrode while electrodes (352a, 352b) together/simultaneously serve as a return electrode.

The bipolar assignments among electrodes (352) as shown in FIGS. 23-27 and described above are not intended to be exhaustive, as any other suitable bipolar assignments may be used. It should also be understood that the bipolar assignments may change during an ablation procedure. For instance, the bipolar assignments depicted in FIG. 23 may be used during one moment of an ablation procedure; and then the bipolar assignments depicted in FIG. 24 may be used during another moment of the same ablation procedure on the same region of tissue. Alternatively, the bipolar assignments for electrodes (352) may change in any other suitable way during the same ablation procedure on the same region of tissue. In some cases, the bipolar assignments for electrodes (352) may change during the same ablation procedure in accordance with a predefined sequence of assignments. In some other cases, the bipolar assignments for electrodes (352) may change during the same ablation procedure on an ad hoc basis, based on real-time feedback. For instance, electrodes (352) may provide tissue impedance sensing, and a control algorithm may be used to change the bipolar assignments for electrodes (352) on an ad hoc basis based on real-time tissue impedance data. In addition, or in the alternative, end effector (350) may include one or more thermocouples and/or other temperature sensing features, and a control algorithm may be used to change the bipolar assignments for electrodes (352) on an ad hoc basis based on real-time temperature data. Alternatively, the bipolar assignments for electrodes (352) may change during the same ablation procedure on an ad hoc basis based on any other suitable criteria. In any of the foregoing scenarios, among others, additional factors that may influence the bipolar assignments for electrodes (352) may include the intended tissue target (e.g., the targeted anatomical structure), the depth or shallowness of the intended lesion, the geometric configuration of the intended lesion, and/or other factors.

### C. Example of Electrical Path Between Electrodes and Guidewire

As noted above with respect to the ground pad (700) in the electrical path depicted in FIG. 22, it is possible to use a second device to provide a return in an electrical path that includes active electrodes (352). As also noted above, the use of ground pad (700) may come at the cost of reduced precision and control in lesions provided via ablation with electrodes (352). This may be due in part to the distance through which the electrical current must travel from electrodes (352) to ground pad (700).

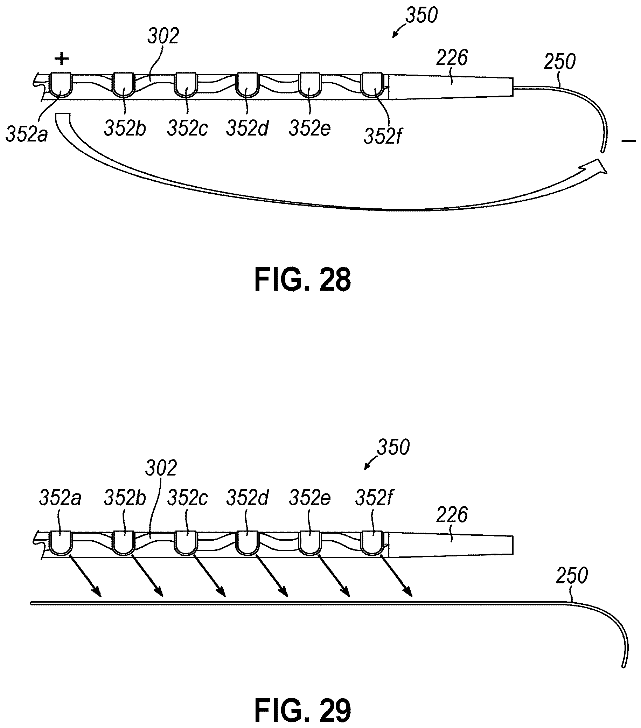

It may therefore be desirable to use a second device to provide a return in an electrical path that includes active electrodes (352) where the second device is substantially closer to electrodes (352), thereby reducing the distance through which the electrical current must travel. To that end, FIG. 28 shows electrodes (352) of end effector (350) being used with guidewire (250) to provide a seventh example of an electrical path. In this example, guidewire (250) is disposed in lumens (236, 227) and protrudes distally from open distal end (228) of tip member (226). Guidewire (250) is also electrically conductive in this example. Thus, guidewire (250) may provide a return path while one or more electrodes (352) are activated with monopolar electrical energy. The electrical energy may flow between the activated electrode(s) (352) and guidewire (250) via adjacent tissue, which may result in ablation of the tissue. While FIG. 28 shows only electrode (352a) serving as an active electrode, any suitable number or position of electrodes (352) may be used to provide an active electrode in cooperation with guidewire (250) as a return electrode.

FIG. 29 shows electrodes (352) of end effector (350) being used with guidewire (250) to provide an eighth example of an electrical path. In this example, guidewire (250) is not positioned within lumens (236, 227) but is instead positioned elsewhere within the patient (PA). For instance, guidewire (250) may be positioned within some other vessel, a chamber of the heart (H), or any other suitable location in the patient (PA). Guidewire (250) may nevertheless be used to provide a return path while one or more electrodes (352) are activated with monopolar electrical energy. The electrical energy may flow between the activated electrode(s) (352) and guidewire (250) via adjacent tissue, which may result in ablation of the tissue.

### D. Example of Electrical Path Between Electrodes and Irrigation Fluid

In some scenarios, it may be beneficial to communicate an irrigation fluid (e.g., saline, etc.) to an ablation instrument and/or to an ablation site during an ablation procedure. For instance, irrigation fluid may prevent arcing during ablation, may prevent buildup of coagulum, may promote electrical conductivity, may prevent overheating, and/or may provide other results. Irrigation fluid may also serve as a virtual electrode. In cases where saline is used as an irrigation fluid that provides a virtual electrode, the salt content of the saline bay be varied to vary the electrical conductivity of the saline, which may in turn affect the performance of the irrigation fluid as a virtual electrode.

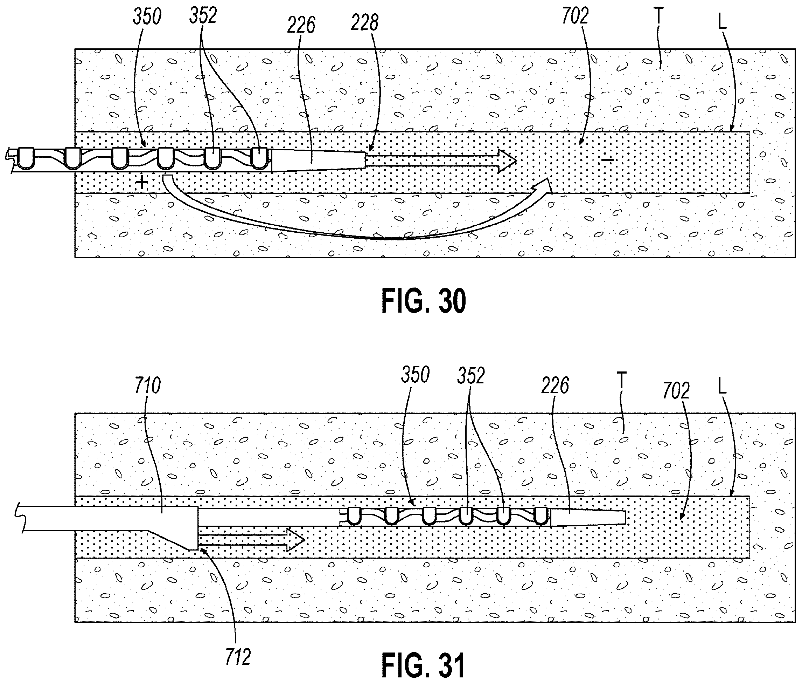

FIGS. 30-31 show examples of how irrigation fluid may be used to provide a virtual electrode that serves as a return path for one or more activated electrodes (352) of end effector (350). Specifically, FIG. 30 shows electrodes (352) of end effector (350) being used with irrigation fluid (700) to provide a ninth example of an electrical path. In this example, end effector (350) is positioned in a lumen (L) defined by tissue (T). By way of example only, the lumen (L) may be within the Vein of Marshall or some other small vessel branching out from the coronary sinus. Irrigation fluid (700) is expelled into the lumen (L) through open distal end (228) of tip member (226) via lumens (236, 227). As noted above, irrigation fluid (700) may be supplied to lumens (236, 227) from fluid source (42) via pump (44), conduit (40), and port (214).

By way of example only, irrigation fluid (700) may be expelled via open distal end (228) at a flow rate of approximately 3 mL/min, at a flow rate greater than approximately 3 mL/min, or at any other suitable flow rate. The expulsion of irrigation fluid (700) into the lumen (L) may eventually result in backflow of irrigation fluid (700) toward electrodes (352). When irrigation fluid (700) has sufficiently reached electrodes (352), one or more electrodes (352) may be activated to provide an active electrode while irrigation fluid (700) serves as a virtual return electrode. The resulting flow of electrical energy may result in ablation of an adjacent region of the tissue (T) defining the lumen (L).

While irrigation fluid (700) may be communicated to the lumen (L) via lumens (236, 227) and open distal end (228) of tip member (226), it may be desirable to instead communicate irrigation fluid (700) to the lumen (L) via some other path. For instance, this may be desirable in cases where the physician (PH) wishes to maintain some other device (e.g., guidewire (250), etc.) within lumens (236, 227), where the presence of that other device within lumens (236, 227) may adversely affect the ability to sufficiently communicate irrigation fluid (700) through lumens (236, 227). FIG. 31 shows electrodes (352) of end effector (350) being used with irrigation fluid (700) to provide a tenth example of an electrical path, where irrigation fluid (700) is communicated through an open distal end (712) of a sheath (710) in which shaft assembly (220) is disposed. In this example open distal end (712) is positioned proximal to end effector (350). In some variations, sheath (710) is in fluid communication with fluid source (42) via pump (44) and conduit (40).

By way of example only, irrigation fluid (700) may be expelled via open distal end (712) at a flow rate of approximately 10 mL/min, at a flow rate greater than approximately 10 mL/min, or at any other suitable flow rate. The expulsion of irrigation fluid (700) into the lumen (L) may eventually result in backflow of irrigation fluid (700) toward electrodes (352). When irrigation fluid (700) has sufficiently reached electrodes (352), one or more electrodes (352) may be activated to provide an active electrode while irrigation fluid (700) serves as a virtual return electrode. The resulting flow of electrical energy may result in ablation of an adjacent region of the tissue (T) defining the lumen (L). While FIGS. 30 and 31 depict irrigation fluid (700) being expelled out through either open distal end (228) of tip member (226) or open distal end (712) of sheath (710), some variations may provide expulsion of irrigation fluid (700) out through both open distal end (228) of tip member (226) and open distal end (712) of sheath (710) (e.g., simultaneously).

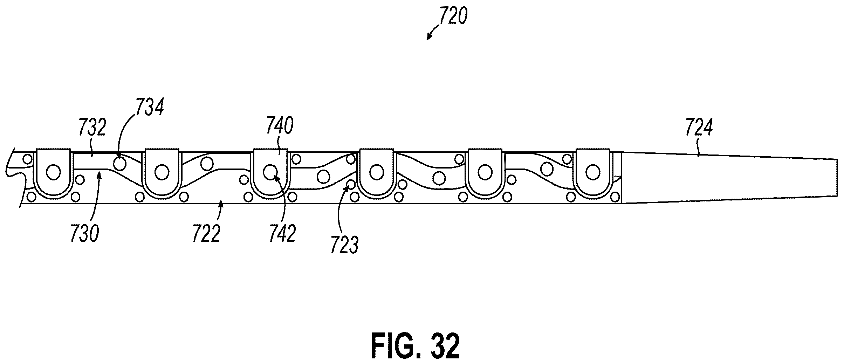

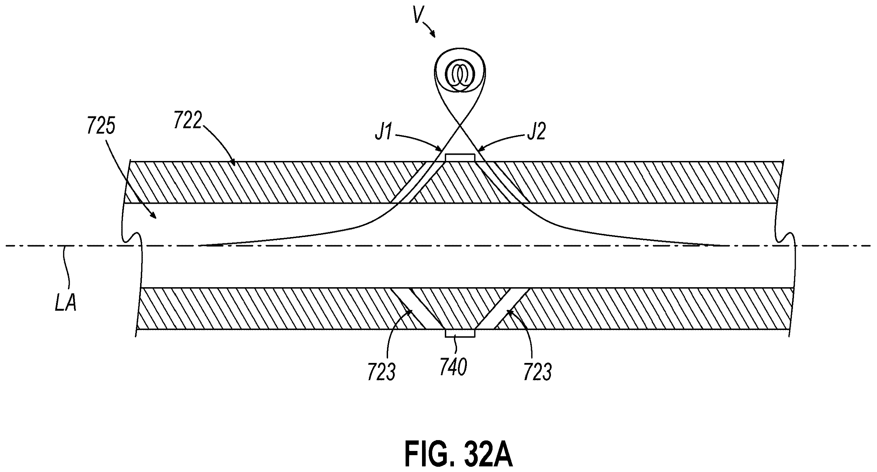

It may also be desirable to expel irrigation fluid (700) out through end effector (350), in addition to expelling irrigation fluid (700) out through open distal end (228) of tip member (226) or out through open distal end (712) of sheath (710); or as an alternative to expelling irrigation fluid (700) out through open distal end (228) of tip member (226) or out through open distal end (712) of sheath (710). To that end, FIG. 32 shows an end effector (720) representing a variation of end effector (350), such that end effector (720) may be provided as a substitute for end effector (350). End effector (720) of this example is configured and operable like end effector (350), except for the differences noted below. End effector (720) is positioned proximal to a tip member (724) that is configured and operable like tip member (226) described above. End effector (720) includes a flex circuit (730) with a flexible substrate (732) wrapped about a catheter body (722). Flex circuit (730) also includes a plurality of electrodes (740) on flexible substrate (732).

Unlike electrodes (352) of end effector (350), electrodes (740) of end effector (720) include irrigation openings (742). Similarly, unlike flexible substrate (302) of flex circuit (300), flexible substrate (732) of flex circuit (730) includes irrigation openings (734) positioned between electrodes (740). Irrigation openings (723) are also formed through catheter body (722) in this example. Irrigation openings (723) may be provided in addition to or in lieu of irrigation openings (734, 742). Similarly, irrigation openings (734) are optional and irrigation openings (742) are optional. In cases where irrigation openings (723) and/or irrigation openings (734) are provided, such irrigation openings (723, 734) may be positioned adjacent to electrodes (740) to promote contact between irrigation fluid expelled out through irrigation openings (723, 734) and electrodes (740). Any suitable number of irrigation openings (723, 734, 742) may be provided, such as 70 irrigation openings (723, 734, 742) or any other suitable number of irrigation openings (723, 734, 742).

Irrigation openings (723, 734, 742) may be in fluid communication with a lumen (not shown) within catheter body (722). This lumen within catheter body (722) may be in further fluid communication with fluid source (42) via pump (44), conduit (40), and port (214).

By way of example only, irrigation fluid (700) may be expelled via irrigation openings (723, 734, 742) at a flow rate of approximately 3 mL/min, at a flow rate greater than approximately 3 mL/min, or at any other suitable flow rate. By way of further example only, irrigation openings (723, 734, 742) may have a diameter of approximately 0.1 mm or any other suitable diameter. With the expelled irrigation fluid (700) contacting electrodes (740), one or more electrodes (740) may be activated to provide an active electrode while irrigation fluid (700) serves as a virtual return electrode. The resulting flow of electrical energy may result in ablation of an adjacent region of the tissue (T) defining the lumen (L).

It should be understood that irrigation fluid (700) may also be expelled out through an open distal end of tip member (724) while also being expelled out through irrigation openings (723, 734, 742). In addition, or in the alternative, irrigation fluid (700) may also be expelled out through open distal end (712) of sheath (710) while also being expelled out through irrigation openings (723, 734, 742). Some variations of end effector (720) may include one or more kinds of irrigation openings (723, 734, 742) while omitting one or more other kinds of irrigation openings (723, 734, 742).

In some versions, the material defining irrigation openings (723, 734, 742) is configured such that irrigation fluid expelled out through irrigation openings (723, 734, 742) is expelled along a path that is obliquely oriented relative to the central longitudinal axis (LA). An example of this is schematically illustrated in FIG. 32A. As shown, irrigation openings (723) adjacent to electrode (740) are oriented obliquely relative to the central longitudinal axis (LA), along respective axes that are oriented toward each other such that one irrigation opening (723) is configured to expel a jet (J1) of irrigation fluid along a path having a distal aspect; while the other irrigation opening (723) is configured to expel a jet (J2) of irrigation fluid along a path having a proximal aspect. Jets (J1, J2) converge to form a vortex (V) of irrigation fluid. This vortex (V) of irrigation fluid is adjacent to electrode (740). While only one set of irrigation openings (723) and adjacent electrode (740) is shown, a similar arrangement may be provided among additional electrodes (740) (if not all electrodes (740)) of the same end effector (720), such that each electrode (740) receives its own local vortex (V) of irrigation fluid. Such local vortices (V) of irrigation fluid may facilitate cooling of electrodes, particularly at the edges of electrodes (740). In some other variations, irrigation fluid at each electrode (740) is expelled only in the direction of jet (J1) or the direction of jet (J2), such that there is no convergence of jets (J1, J2). Alternatively, irrigation fluid may be expelled in any other suitable fashion.

### E. Example of Electrical Path Between Electrodes and Other Ablation Instrument

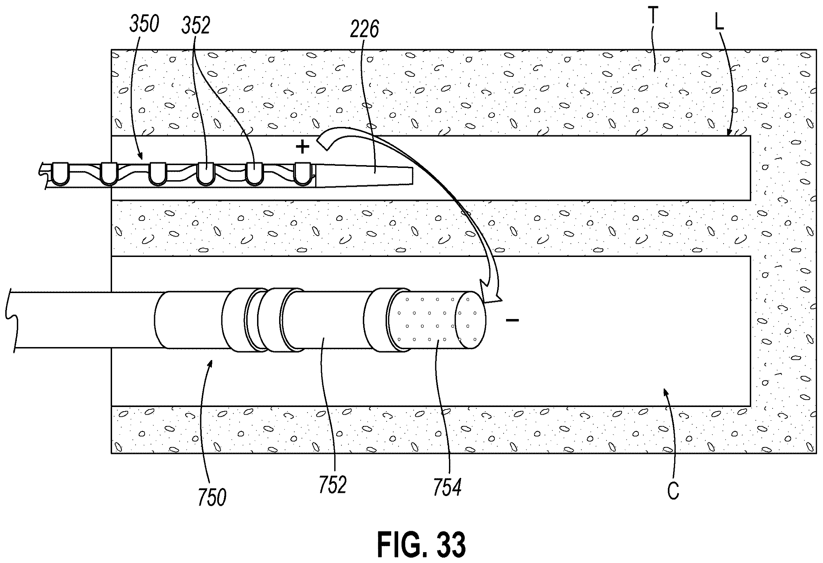

There may be some scenarios where two different kinds of ablation instruments are positioned in the same patient (PA) simultaneously. This may include catheter assembly (200) and another instrument that has at least one ablation electrode. In such scenarios, it may be desirable to provide a bipolar coupling between at least one electrode (352) of end effector (350) and at least one electrode of the other ablation instrument. To that end, FIG. 33 shows electrodes (352) of end effector (350) being used with another ablation instrument (750) to provide an eleventh example of an electrical path. In this example, end effector (350) is positioned in a lumen (L) defined by tissue (T). By way of example only, the lumen (L) may be within the Vein of Marshall or some other small vessel branching out from the coronary sinus.

The other ablation instrument (750) is positioned in a larger cavity (C), such as a chamber of the heart (H) or a space external to the heart (H). Instrument (750) may comprise a sheath or some other delivery instrument, another catheter, and/or any other suitable kind of instrument. In the present example, instrument (750) includes a shaft (752) with a distal tip electrode (754). Alternatively, instrument (750) may have any other suitable kind of electrically conductive feature at any other suitable location along shaft (752). In the example shown in FIG. 33, electrode (754) of instrument (750) is used to provide a return path while one or more electrodes (352) are activated with monopolar electrical energy. The electrical energy may flow between the activated electrode(s) (352) and electrode (754) via adjacent tissue (T), which may result in ablation of the tissue (T). While not shown in FIG. 33, irrigation fluid (700) may be provided in the lumen (L) and/or in the chamber (C) during this process. In some cases, instrument (750) may also be used to ablate tissue and/or provide EP mapping via electrode (754) and/or via other features in the chamber (C). Such ablation by instrument (750) in the chamber (C) may be provided within the same procedure where end effector (350) is used to provide ablation in the lumen (L).

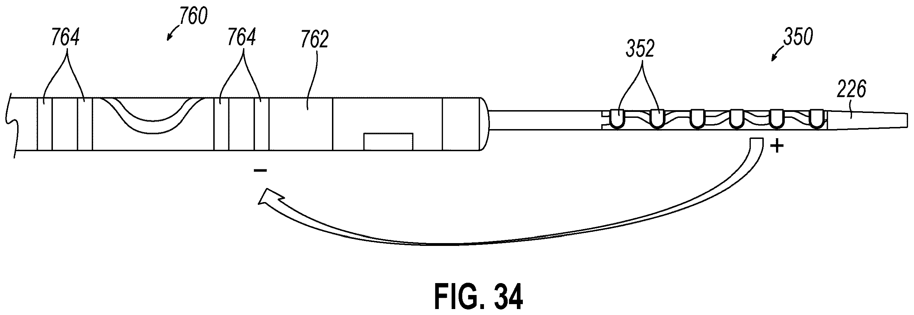

FIG. 34 shows electrodes (352) of end effector (350) being used with another ablation instrument (760) to provide a twelfth example of an electrical path. In this example, shaft assembly (220) is disposed within a lumen of instrument (760), with end effector (350) being positioned distally relative to instrument (760). Instrument (760) may comprise a sheath or some other delivery instrument, another catheter, and/or any other suitable kind of instrument. Instrument (760) of this example includes a shaft (762) with a set of ring electrodes (764). Alternatively, instrument (760) may have any other suitable kind of electrically conductive feature at any other suitable location along shaft (762). In the example shown in FIG. 33, one or more electrodes (764) of instrument (760) are used to provide a return path while one or more electrodes (352) are activated with monopolar electrical energy. The electrical energy may flow between the activated electrode(s) (352) and electrode(s) (764) via adjacent tissue, which may result in ablation of the tissue.

In some cases, instrument (760) is advanced along a larger lumen (e.g., within the coronary sinus) and is used to ablate tissue defining the larger lumen (e.g., via activation of electrodes (764)). While instrument (760) is held in place within that larger lumen, end effector (350) may be advanced into a smaller lumen (e.g., in the Vein of Marshall) branching off from the larger lumen. One or more electrode(s) (352) may then be used with one or more electrode(s) (764) to form a bipolar pair and thereby ablate tissue defining the smaller lumen. Alternatively, end effector (350) may be used with another instrument (760) in any other suitable fashion.

### V. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus, comprising: (a) an elongate shaft assembly, the shaft assembly having a proximal portion and a distal portion, the distal portion having a distal end, the distal portion being flexible, the shaft assembly having an outer diameter; (b) an end effector positioned at the distal end of the shaft assembly, the end effector including one or more electrodes; and (c) a flexible circuit including: (i) a flexible substrate, the flexible substrate including: (A) a proximal portion, the proximal portion of the flexible substrate being positioned proximal to the end effector, (B) an intermediate portion, the intermediate portion extending along the distal portion of the shaft assembly, the intermediate portion being positioned proximal to the end effector, and (C) a distal portion, and (ii) a first trace extending along at least the intermediate portion of the flexible substrate, a portion of the first trace defining a first position sensor having a first position sensor length extending along a first portion of a length of the distal portion of the shaft assembly, the first position sensor being configured to generate a signal in the presence of an alternating magnetic field, the first position sensor being further configured to flex with the distal portion of the shaft assembly, the first position sensor length being at least approximately 10 times greater than the outer diameter of the shaft assembly.

### Example 2

The apparatus of Example 1, the proximal portion of the shaft assembly being rigid.

### Example 3

The apparatus of Example 2, the shaft assembly further comprising an intermediate portion providing a gradual transition in flexibility from the rigid proximal portion flexible distal portion.

### Example 4

The apparatus of any of Examples 1 through 3, the end effector including a plurality of electrodes, the electrodes being longitudinally spaced apart from each other along the distal end of the shaft assembly.

### Example 5

The apparatus of Example 4, the electrodes being operable to apply electrical energy to tissue to thereby ablate the tissue.

### Example 6

The apparatus of Example 5, the electrodes being operable to provide pulsed field ablation to tissue.

### Example 7

The apparatus of any of Examples 1 through 6, the one or more electrodes being positioned on the distal portion of the flexible substrate.

### Example 8

The apparatus of any of Examples 1 through 7, the intermediate portion comprising a first longitudinally extending segment and a second longitudinally extending segment, the first longitudinally extending segment being angularly spaced apart from the second longitudinally extending segment.

### Example 9

The apparatus of Example 8, the first longitudinally extending segment being angularly spaced apart from the second longitudinally extending segment by approximately 180 degrees about a central longitudinal axis.

### Example 10

The apparatus of any of Examples 8 through 9, the first longitudinally extending segment having a serpentine configuration.

### Example 11

The apparatus of Example 10, the second longitudinally extending segment having a serpentine configuration.

### Example 12

The apparatus of any of Examples 8 through 11, the portion of the first trace defining the first position sensor extending along the first longitudinally extending segment and along the second longitudinally extending segment.

### Example 13

The apparatus of Example 12, the flexible substrate further comprising an angularly extending segment joining the first longitudinally extending segment with the second longitudinally extending segment, the portion of the first trace defining the first position sensor further extending along the angularly extending segment.

### Example 14

The apparatus of any of Examples 1 through 13, the portion of the first trace defining the first position sensor defining a coil shape.

### Example 15

The apparatus of any of Examples 1 through 14, the proximal portion of the flexible substrate including a tail portion configured to wrap helically about the distal portion of the shaft assembly.

### Example 16

The apparatus of any of Examples 1 through 15, the flexible circuit further including one or more electrical contact pads positioned on the proximal portion of the flexible substrate.

### Example 17

The apparatus of any of Examples 1 through 16, the portion of the first trace defining a position sensor further extending along the distal portion of the flexible substrate.

### Example 18

The apparatus of any of Examples 1 through 16, the portion of the first trace defining a position sensor further extending along the end effector.

### Example 19

The apparatus of any of Examples 1 through 18, further comprising a tip member secured to the distal portion of the shaft assembly, distal to the end effector.

### Example 20

The apparatus of Example 19, the tip member having greater flexibility than the distal portion of the shaft assembly.

### Example 21

The apparatus of Example 21, the distal portion of the shaft assembly including a braided internal structure, the tip member lacking a braided internal structure such that the lack of a braided internal structure provides greater flexibility to the tip member as compared to the flexibility of the distal portion of the shaft assembly.

### Example 22

The apparatus of any of Examples 19 through 21, the shaft assembly and the tip member together defining a lumen, the lumen distally terminating at an open distal end of the tip member.

### Example 23

The apparatus of Example 22, the lumen being sized to receive a guidewire.

### Example 24

The apparatus of any of Examples 22 through 23, the lumen being configured to communicate fluid through the elongate shaft assembly and out through the open distal end of the tip member.

### Example 25

The apparatus of any of Examples 1 through 24, the shaft assembly having a total length extending from a proximal end of the shaft assembly to the distal end of the shaft assembly, the first position sensor defined by the portion of the first trace having a total length extending from a proximal end of the first position sensor to a distal end of the first position sensor, the total length of the first position sensor being at least approximately 5% of the total length of the shaft assembly.

### Example 26

The apparatus of any of Examples 1 through 25, the flexible circuit further including a second trace extending along at least the intermediate portion of the flexible substrate, a portion of the second trace defining a second position sensor extending along a second portion of a length of the distal portion of the shaft assembly, the second position sensor being configured to generate a signal in the presence of an alternating magnetic field.

### Example 27

The apparatus of Example 26, the second position sensor being positioned distally in relation to the first position sensor.

### Example 28

The apparatus of Example 26, the second position sensor being longitudinally coextensive with the first position sensor.

### Example 29

The apparatus of any of Examples 26 through 28, the second position sensor being angularly offset from the first position sensor about a central longitudinal axis.

### Example 30

The apparatus of Example 29, the second position sensor being angularly offset from the first position sensor about a central longitudinal axis by approximately 90 degrees.

### Example 31

The apparatus of any of Examples 26 through 30, the first position sensor comprising a first set of loop segments, the second position sensor comprising a second set of loop segments.

### Example 32

The apparatus of Example 31, the loop segments of the first set of loop segments being interleaved with the loop segments of the second set.

### Example 33

The apparatus of any of Examples 26 through 32, the second trace being electrically isolated relative to the first trace.

### Example 34

The apparatus of any of Examples 26 through 33, the flexible substrate further including an angularly extending segment providing an angular offset and a longitudinal offset between the first position sensor and the second position sensor.

### Example 35

The apparatus of any of Examples 1 through 34, the outer diameter of the shaft assembly having a value ranging from approximately 0.67 mm to approximately 1.33 mm.

### Example 36

The apparatus of any of Examples 1 through 35, the apparatus having an inner diameter ranging from approximately 0.25 mm to approximately 0.45 mm.

### Example 37

The apparatus of any of Examples 1 through 36, the shaft assembly having a length ranging from approximately 150 cm to approximately 200 cm.

### Example 38

The apparatus of any of Examples 1 through 37, the distal portion of the shaft assembly having a length ranging from approximately 30 cm to approximately 75 cm.

### Example 39

The apparatus of any of Examples 1 through 28, the end effector being sized to fit within a vessel branching off from a coronary sinus of a patient.

### Example 40

The apparatus of Example 39, the end effector being sized to fit within a Vein of Marshall of a patient.

### Example 41

An apparatus, comprising: (a) an elongate shaft assembly, the shaft assembly having a total length extending from a proximal end of the shaft assembly to the distal end of the shaft assembly; (b) an end effector positioned at the distal end of the shaft assembly, the end effector including one or more electrodes; and (c) a flexible circuit including: (i) a flexible substrate, the flexible substrate including: (A) a proximal portion, the proximal portion of the flexible substrate being positioned proximal to the end effector, (B) an intermediate portion, the intermediate portion extending along the distal portion of the shaft assembly, the intermediate portion being positioned proximal to the end effector, and (C) a distal portion, and (ii) a trace extending along at least the intermediate portion of the flexible substrate, a portion of the trace defining a position sensor having a position sensor length extending along a portion of a length of the distal portion of the shaft assembly, the position sensor being configured to generate a signal in the presence of an alternating magnetic field, the position sensor being further configured to flex with the distal portion of the shaft assembly, the position sensor length being at least approximately 5% of the total length of the shaft assembly.

### Example 42

The apparatus of Example 41, the shaft assembly having an outer diameter, the position sensor length being at least approximately 25 times greater than the outer diameter of the shaft assembly.

### Example 43

An apparatus, comprising: (a) an elongate shaft assembly, the shaft assembly having a total length extending from a proximal end of the shaft assembly to the distal end of the shaft assembly; (b) an end effector positioned at the distal end of the shaft assembly, the end effector including one or more electrodes; and (c) a flexible circuit including: (i) a flexible substrate, the flexible substrate including: (A) a proximal portion, the proximal portion of the flexible substrate being positioned proximal to the end effector, (B) an intermediate portion, the intermediate portion extending along the distal portion of the shaft assembly, the intermediate portion being positioned proximal to the end effector, and (C) a distal portion, and (ii) a trace extending along at least the intermediate portion of the flexible substrate, a portion of the trace defining a position sensor having an effective position sensor length extending along a portion of a length of the distal portion of the shaft assembly, the position sensor having an effective position sensor width extending transversely relative to the length of the distal portion of the shaft assembly, the position sensor being configured to generate a signal in the presence of an alternating magnetic field, the position sensor being further configured to flex with the distal portion of the shaft assembly, the effective position sensor length and the effective position sensor width together defining an area along a two-dimensional plane, the area being configured to change with one or both of the effective position sensor length or the effective position sensor width as the position sensor flexes with the distal portion of the shaft assembly, the position sensor being further configured such that the area will not get smaller than approximately 150 mm² when the position sensor flexes with the distal portion of the shaft assembly.

### Example 44

The apparatus of Example 43, the position sensor effective length being at least approximately 5% of the total length of the shaft assembly.

### Example 45

The apparatus of any of Examples 43 through 44, the shaft assembly having an outer diameter, the position sensor effective length being at least approximately 25 times greater than the outer diameter of the shaft assembly.

### Example 46

An apparatus, comprising: (a) an elongate shaft assembly, the shaft assembly having a proximal portion and a distal portion, the distal portion having a distal end, the distal portion being flexible; (b) an end effector positioned at the distal end of the shaft assembly, the end effector including one or more electrodes; and (c) a flexible circuit including: (i) a flexible substrate, the flexible substrate including: (A) a proximal portion, the proximal portion of the flexible substrate being positioned proximal to the end effector, (B) an intermediate portion, the intermediate portion extending along the distal portion of the shaft assembly, the intermediate portion being positioned proximal to the end effector, the intermediate portion comprising a first longitudinally extending segment and a second longitudinally extending segment, the first longitudinally extending segment being angularly spaced apart from the second longitudinally extending segment, and (C) a distal portion, and (ii) a trace extending along the first longitudinally extending segment and along the second longitudinally extending segment, the trace defining a position sensor extending along a portion of a length of the distal portion of the shaft assembly, the position sensor being configured to generate a signal in the presence of an alternating magnetic field.

### Example 47

The apparatus of Example 79, the position sensor being further configured to flex with the distal portion of the shaft assembly.

### Example 48

The apparatus of any of Examples 79 through 80, the first longitudinally extending segment being angularly spaced apart from the second longitudinally extending segment by approximately 180 degrees about a central longitudinal axis.

### Example 49

The apparatus of any of Examples 79 through 81, the first longitudinally extending segment having a serpentine configuration.

### Example 50

The apparatus of Example 82, the second longitudinally extending segment having a serpentine configuration.

### Example 51

The apparatus of Example 83, the serpentine configuration of the first longitudinally extending segment defining a first plurality of peaks, the serpentine configuration of the first longitudinally extending segment defining a second plurality of peaks.

### Example 52

The apparatus of Example 84, the peaks of the first plurality of peaks being positioned to coincide with the peaks of the second plurality of peaks along a dimension transversely oriented relative to a longitudinal dimension.

### Example 53

The apparatus of any of Examples 79 through 85, the flexible substrate further comprising an angularly extending segment joining the first longitudinally extending segment with the second longitudinally extending segment, the portion of the trace defining the first position sensor further extending along the angularly extending segment.

### Example 54

The apparatus of any of Examples 79 through 86, the portion of the trace defining the position sensor defining a coil shape.

### Example 55

The apparatus of any of Examples 79 through 87, the intermediate portion further comprising a plurality of web segments, the web segments being longitudinally spaced apart from each other, the web segments joining the first longitudinally extending segment with the second longitudinally extending segment.

### Example 56

A method comprising: inserting an elongate shaft assembly into an anatomical structure within a cardiovascular system of a patient, the elongate shaft assembly having a distal portion with an end effector, the end effector having a plurality of electrodes extending angularly about the distal portion of the shaft assembly and spaced longitudinally apart from each other along the distal portion of the shaft assembly, the shaft assembly further including a tip member positioned distal to the end effector, the tip member being more flexible than the distal portion of the shaft assembly; selecting one or more electrodes of the plurality of electrodes to activate; and activating the one or more selected electrodes to apply electrical energy to tissue to thereby ablate the tissue.

### Example 57

The method of Example 56, the anatomical passageway comprising a vessel branching off from a coronary sinus.

### Example 58

The method of Example 57, the anatomical passageway comprising a Vein of Marshall.

### Example 59

The method of any of Examples 56 through 58, inserting the elongate shaft assembly into the anatomical structure including advancing the elongate shaft assembly along a guidewire, the guidewire being slidably disposed within the elongate shaft assembly.

### Example 60

The method of Example 59, activating the one or more selected electrodes to apply electrical energy to tissue to thereby ablate the tissue comprising utilizing the guidewire as a return path for the electrical energy.

### Example 61

The method of any of Examples 56 through 60, activating the one or more selected electrodes to apply electrical energy to tissue to thereby ablate the tissue comprising utilizing a ground pad as a return path for the electrical energy.

### Example 62

The method of Example 61, the ground pad comprising a patch adhered to the patient.

### Example 63

The method of any of Examples 56 through 62, selecting one or more electrodes of the plurality of electrodes comprising selecting a first single electrode of the plurality of electrodes to serve as an active electrode.

### Example 64

The method of any of Examples 56 through 62, selecting one or more electrodes of the plurality of electrodes comprising selecting a first set of electrodes of the plurality of electrodes to collectively serve as an active electrode.

### Example 65

The method of any of Examples 56 through 64, selecting one or more electrodes of the plurality of electrodes comprising selecting a first single electrode of the plurality of electrodes to serve as a return electrode.

### Example 66

The method of any of Examples 56 through 64, selecting one or more electrodes of the plurality of electrodes comprising selecting a first set of electrodes of the plurality of electrodes to collectively serve as a return electrode.

### Example 67

The method of any of Examples 56 through 66, activating the one or more selected electrodes to apply electrical energy to tissue to thereby ablate the tissue comprising utilizing a separate instrument to provide a return path for the electrical energy, the separate instrument being positioned within the patient.

### Example 68

The method of Example 67, the separate instrument comprising a guidewire.

### Example 69

The method of Example 68, the guidewire being positioned in a different location within the patient such that the guidewire is not positioned within the anatomical structure in which the elongate shaft assembly is disposed.

### Example 70

The method of Example 67, the separate instrument comprising an ablation instrument having an electrode.

### Example 71

The method of Example 70, the ablation instrument being positioned in a different location within the patient such that the ablation instrument is not positioned within the anatomical structure in which the elongate shaft assembly is disposed.

### Example 72

The method of Example 71, the ablation instrument being positioned within a chamber of the heart or in a pulmonary vein.

### Example 73

The method of any of Examples 71 through 72, the ablation instrument defining a lumen, the elongate shaft assembly being slidably disposed within the lumen of the ablation instrument.

### Example 74

The method of Example 73, the ablation instrument being positioned with in a coronary sinus, the elongate shaft assembly being positioned within a vessel branching off from the coronary sinus.

### Example 75

The method of any of Examples 56 through 74, further comprising communicating irrigation fluid to the anatomical structure.

### Example 76

The method of Example 75, activating the one or more selected electrodes to apply electrical energy to tissue to thereby ablate the tissue further comprising using the irrigation fluid to provide a return path for the electrical energy.

### Example 77

The method of any of Examples 74 through 76, communicating irrigation fluid to the anatomical structure comprising ejecting irrigation fluid out through an open distal end of the tip member.

### Example 78

The method of any of Examples 76 through 77, the elongate shaft assembly being positioned within a sheath, communicating irrigation fluid to the anatomical structure comprising ejecting irrigation fluid out through an open distal end of the sheath.

### Example 79

The method of Example 78, the open distal end of the sheath being positioned proximal to the end effector.

### Example 80

The method of any of Examples 76 through 79, communicating irrigation fluid to the anatomical structure comprising ejecting irrigation fluid out through lateral openings formed in the end effector.

### Example 81

The method of Example 75, at least one of the lateral openings being positioned in at least one electrode of the plurality of electrodes.

### Example 82

The method of any of Examples 80 through 81, the lateral openings being oriented obliquely relative to a central longitudinal axis of the shaft assembly, such that the ejected irrigation fluid is ejected in jets oriented obliquely relative to a central longitudinal axis of the shaft assembly.

### Example 83

The method of Example 82, at least some of the jets forming vortices of irrigation fluid adjacent to the plurality of electrodes.

### Example 84

The method of Example 83, at least some of the jets being oriented toward each other to form the vortices of irrigation fluid adjacent to the plurality of electrodes.

### Example 85

The method of any of Examples 56 through 84, activating the one or more selected electrodes to apply electrical energy to tissue to thereby ablate the tissue comprising applying radiofrequency energy to tissue.

### Example 86

The method of any of Examples 56 through 84, activating the one or more selected electrodes to apply electrical energy to tissue to thereby ablate the tissue comprising providing pulsed field ablation to the tissue.

### Example 87

The method of any of Examples 56 through 86, further comprising processing a signal from a position sensor, the signal indicating a real-time position of the end effector.

### Example 88

The method of Example 87, position sensor comprising a coil formed by part of a flexible circuit, the coil flexing with the distal portion of the elongate shaft assembly during insertion of the elongate shaft assembly into the anatomical structure.

### Example 89

An apparatus, comprising: (a) an elongate shaft assembly, the shaft assembly having a proximal portion and a distal portion, the distal portion having a distal end, the distal portion being flexible; (b) an end effector positioned at the distal end of the shaft assembly; (c) a flexible circuit, the flexible circuit including: (i) a flexible substrate, (ii) at least one position sensor supported by the flexible substrate, the at least one position sensor being configured to provide a signal indicating a position of the end effector, and (iii) a plurality of electrodes supported by the flexible substrate, the electrodes of the plurality of electrodes being longitudinally spaced apart from each other along the end effector; and (d) a processor electrically coupled with the flex circuit, the processor being configured to: (i) determine an arrangement of a bipolar pair of electrodes based on one or more criteria, (ii) select one or more electrodes of the plurality of electrodes based on the determination, and (iii) activate the one or more selected electrodes to apply electrical energy to tissue to thereby ablate the tissue.

### Example 90

The apparatus of Example 89, the one or more criteria including one or more criteria selected from the group consisting of a targeted anatomical structure, a depth of intended lesion, and a geometric configuration of an intended lesion.

### Example 91

A method comprising: inserting an elongate shaft assembly of a first instrument into a first anatomical structure within a cardiovascular system of a patient, the elongate shaft assembly having a distal portion with an end effector, the end effector having a plurality of electrodes extending angularly about the distal portion of the shaft assembly and spaced longitudinally apart from each other along the distal portion of the shaft assembly, the shaft assembly further including a tip member positioned distal to the end effector, the tip member being more flexible than the distal portion of the shaft assembly; inserting a second instrument into a second anatomical structure within the patient, the second instrument having at least one electrically conductive feature; selecting one or more electrodes of the plurality of electrodes to activate; and activating the one or more selected electrodes to apply electrical energy to tissue to thereby ablate the tissue, the at least one electrically conductive feature of the second instrument providing a return path for the electrical energy.

### Example 92

The method of Example 91, the second anatomical structure being within the cardiovascular system of a patient.

### Example 93

The method of Example 92, the second anatomical structure comprising a chamber of a heart of the patient.

### Example 94

The method of any of Examples 91 through 93, the first anatomical structure comprising a vessel branching out from a coronary sinus of the patient.

### Example 95

The method of any of Examples 91 through 94, the second instrument comprising a guidewire.

### Example 96.

The method of any of Examples 91 through 94, the second instrument comprising an ablation catheter.

### Example 97

The method of any of Examples 91 through 96, the at least one electrically conductive feature of the second instrument comprising a distal tip electrode.

### Example 98

The method of any of Examples 91 through 97, the first instrument and the second instrument being separated by tissue between the first anatomical structure and the second anatomical structure.

### Example 99

The method of any of Examples 91 through 98, the first anatomical structure comprising a first vessel, the second anatomical structure comprising a second vessel, the first vessel branching off of the second vessel.

### Example 100

The method of Example 99, the first instrument being slidably disposed within the second instrument.

### Example 101

An apparatus, comprising: (a) an elongate shaft assembly, the elongate shaft assembly including a distal portion, the distal portion including: (i) an end effector, the end effector having a flex circuit with a plurality of electrodes extending angularly about the distal portion of the shaft assembly and spaced longitudinally apart from each other along the distal portion of the shaft assembly, and (ii) a tip member positioned distal to the end effector, the tip member being more flexible than the distal portion of the shaft assembly; and (b) a processor electrically coupled with the flex circuit, the processor being configured to execute the following steps: (i) selecting one or more electrodes of the plurality of electrodes to activate, and (ii) activating the one or more selected electrodes to apply electrical energy to tissue to thereby ablate the tissue via pulsed field ablation.

### Example 102

The apparatus of Example 101, the distal portion being sized to fit in a vessel branching off from a coronary sinus.

### Example 103

The apparatus of Example 102, the vessel comprising a Vein of Marshall.

### Example 104

The apparatus of any of Examples 101 through 103, further comprising a guidewire, the guidewire being configured to slidably fit within the elongate shaft assembly.

### Example 105

The apparatus of Example 104, the processor being further configured to activate the one or more selected electrodes to apply electrical energy to tissue to thereby ablate the tissue while utilizing the guidewire as a return path for the electrical energy.

### Example 106

The apparatus of any of Examples 101 through 105, the processor being further configured to activate the one or more selected electrodes to apply electrical energy to tissue to thereby ablate the tissue while utilizing a ground pad as a return path for the electrical energy.

### Example 107

The apparatus of any of Examples 101 through 106, the processor being further configured to select a single electrode of the plurality of electrodes to serve as an active electrode.

### Example 108

The apparatus of any Examples 101 through 107, the processor being further configured to select a set of electrodes of the plurality of electrodes to collectively serve as an active electrode.

### Example 109

The apparatus of any of Examples 101 through 108, the processor being further configured to select a single electrode of the plurality of electrodes to serve as a return electrode.

### Example 120

The apparatus of any of Examples 101 through 109, the processor being further configured to select a set of electrodes of the plurality of electrodes to collectively serve as a return electrode.

### Example 121

The apparatus of any of Examples 101 through 120, further comprising a separate instrument, the processor being further configured to activate the one or more selected electrodes to apply electrical energy to tissue to thereby ablate the tissue while utilizing the separate instrument as a return path for the electrical energy.

### Example 122

The apparatus of Example 121, the separate instrument comprising a guidewire, the processor being further configured to activate the one or more selected electrodes to apply electrical energy to tissue to thereby ablate the tissue while utilizing the guidewire as a return path for the electrical energy when the guidewire is positioned in an anatomical structure different from an anatomical structure in which the distal portion of the elongate shaft assembly is positioned.

### Example 123

The apparatus of Example 121, the separate instrument comprising an ablation instrument having an electrode.

### Example 124

The apparatus of Example 123, the ablation instrument defining a lumen, the elongate shaft assembly being configured to slidably fit within the lumen of the ablation instrument.

### Example 125

The apparatus of any of Examples 101 through 124, the elongate shaft assembly being further configured to communicate irrigation fluid to an anatomical structure.

### Example 126

The apparatus of Example 125, processor being further configured to activate the one or more selected electrodes to apply electrical energy to tissue to thereby ablate the tissue while utilizing the irrigation fluid as a return path for the electrical energy.

### Example 127

The apparatus of any of Examples 124 through 125, the end effector further including lateral openings, the lateral openings being configured to emit the irrigation fluid.

### VI. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) an elongate shaft assembly, the elongate shaft assembly including a distal portion, the distal portion including:
(i) an end effector, the end effector having a flex circuit with a plurality of electrodes extending angularly about the distal portion of the shaft assembly and spaced longitudinally apart from each other along the distal portion of the shaft assembly, and
(ii) a tip member positioned distal to the end effector, the tip member being more flexible than the distal portion of the shaft assembly; and
(b) a processor electrically coupled with the flex circuit, the processor being configured to execute the following steps:
(i) selecting one or more electrodes of the plurality of electrodes to activate, and
(ii) activating the one or more selected electrodes to apply electrical energy to tissue to thereby ablate the tissue via pulsed field ablation.

2. The apparatus of claim 1, the distal portion being sized to fit in a vessel branching off from a coronary sinus, optionally the vessel comprising a Vein of Marshall.

3. The apparatus of any preceding claim, further comprising a guidewire, the guidewire being configured to slidably fit within the elongate shaft assembly.

4. The apparatus of claim 3, the processor being further configured to activate the one or more selected electrodes to apply electrical energy to tissue to thereby ablate the tissue while utilizing the guidewire as a return path for the electrical energy.

5. The apparatus of any preceding claim, the processor being further configured to activate the one or more selected electrodes to apply electrical energy to tissue to thereby ablate the tissue while utilizing a ground pad as a return path for the electrical energy.

6. The apparatus of any preceding claim, the processor being further configured to (i) select a single electrode of the plurality of electrodes to serve as an active electrode or (ii) select a set of electrodes of the plurality of electrodes to collectively serve as an active electrode.

7. The apparatus of any preceding claim, the processor being further configured to (i) select a single electrode of the plurality of electrodes to serve as a return electrode or (ii) select a set of electrodes of the plurality of electrodes to collectively serve as a return electrode.

8. The apparatus of any preceding claim, further comprising a separate instrument, the processor being further configured to activate the one or more selected electrodes to apply electrical energy to tissue to thereby ablate the tissue while utilizing the separate instrument as a return path for the electrical energy.

9. The apparatus of claim 8, the separate instrument comprising a guidewire, the processor being further configured to activate the one or more selected electrodes to apply electrical energy to tissue to thereby ablate the tissue while utilizing the guidewire as a return path for the electrical energy when the guidewire is positioned in an anatomical structure different from an anatomical structure in which the distal portion of the elongate shaft assembly is positioned.

10. The apparatus of claim 8, the separate instrument comprising an ablation instrument having an electrode.

11. The apparatus of claim 10, the ablation instrument defining a lumen, the elongate shaft assembly being configured to slidably fit within the lumen of the ablation instrument.

12. The apparatus of any preceding claim, the elongate shaft assembly being further configured to communicate irrigation fluid to an anatomical structure.

13. The apparatus of claim 12, processor being further configured to activate the one or more selected electrodes to apply electrical energy to tissue to thereby ablate the tissue while utilizing the irrigation fluid as a return path for the electrical energy and/or the end effector further including lateral openings, the lateral openings being configured to emit the irrigation fluid.

14. An apparatus, comprising:
(a) an elongate shaft assembly, the shaft assembly having a proximal portion and a distal portion, the distal portion having a distal end, the distal portion being flexible;
(b) an end effector positioned at the distal end of the shaft assembly;
(c) a flexible circuit, the flexible circuit including:
(i) a flexible substrate,
(ii) at least one position sensor supported by the flexible substrate, the at least one position sensor being configured to provide a signal indicating a position of the end effector, and
(iii) a plurality of electrodes supported by the flexible substrate, the electrodes of the plurality of electrodes being longitudinally spaced apart from each other along the end effector; and
(d) a processor electrically coupled with the flex circuit, the processor being configured to:
(i) determine an arrangement of a bipolar pair of electrodes based on one or more criteria,
(ii) select one or more electrodes of the plurality of electrodes based on the determination, and
(iii) activate the one or more selected electrodes to apply electrical energy to tissue to thereby ablate the tissue.

15. The apparatus of claim 14, the one or more criteria including one or more criteria selected from the group consisting of a targeted anatomical structure, a depth of intended lesion, and a geometric configuration of an intended lesion.
